# EUROPEAN PATENT SPECIFICATION

(11) **EP 4 078 178 B1**
(45) Date of publication and mention of the grant of the patent: **23.10.2024**
(21) Application number: 20820409.9
(22) Date of filing: 09.12.2020
(51) Int. Cl.: G01N 33/50, G01N 33/533

(54) **REVERSIBLE CELL DETECTION VIA MHC WITH CONJUGATES HAVING AN ENZYMATICALLY CLEAVABLE DETECTION MOIETY**
REVERSIBLE ZELLDETEKTION ÜBER MHC MIT KONJUGATEN MIT EINER ENZYMATISCH SPALTBAREN DETEKTIONSEINHEIT
DÉTECTION DE CELLULES RÉVERSIBLES VIA MHC AVEC DES CONJUGUÉS POSSÉDANT UNE FRACTION DE DÉTECTION CLIVABLE PAR VOIE ENZYMATIQUE

(30) Priority: 20.12.2019 EP 19218591
(43) Date of publication of application: 26.10.2022
(73) Proprietor: Miltenyi Biotec B.V. & Co. KG, 51429 Bergisch Gladbach (DE)
(72) Inventor: A, A, 51429 Bergisch Gladbach (DE); BRIEDEN, Jennifer, 51429 Bergisch Gladbach (DE); PANKRATZ, Jennifer, 51429 Bergisch Gladbach (DE); EVARISTO, Cesar, 51429 Bergisch Gladbach (DE); NÖLLE, Volker, 51429 Bergisch Gladbach (DE); WIECZOREK, Marek, 51429 Bergisch Gladbach (DE)
(74) Representative: Kisters, Michael Marcus
(86) International application number: PCT/EP2020/085175
(87) International publication number: WO 2021/122185

(56) References cited:
- EP-A1- 3 037 821
- WO-A1-2010/037397
- WO-A2-02/072631
- WO-A2-2009/039854
- WO-A2-2010/037395
- WO-A2-2016/198932
- US-A1- 2005 003 431
- O'CALLAGHAN C A ET AL: "BirA enzyme: production and application in the study of membrane receptor-ligand interactions bysite-specific biotinylation", ANALYTICAL BIOCHEMISTRY, ACADEMIC PRESS, AMSTERDAM, NL, vol. 266, no. 1, 1 January 1999 (1999-01-01), pages 9 - 15, XP002107534, ISSN: 0003-2697, DOI: 10.1006/ABIO.1998.2930
- G. DOLTON ET AL: "Comparison of peptide-major histocompatibility complex tetramers and dextramers for the identification of antigen-specific T cells", CLINICAL AND EXPERIMENTAL IMMUNOLOGY, vol. 177, no. 1, 9 June 2014 (2014-06-09), GB, pages 47 - 63, XP055360855, ISSN: 0009-9104, DOI: 10.1111/cei.12339
- LINDA WOOLDRIDGE ET AL: "Interaction between the CD8 Coreceptor and Major Histocompatibility Complex Class I Stabilizes T Cell Receptor-Antigen Complexes at the Cell Surface", JOURNAL OF BIOLOGICAL CHEMISTRY, vol. 280, no. 30, 29 July 2005 (2005-07-29), US, pages 27491 - 27501, XP055701008, ISSN: 0021-9258, DOI: 10.1074/jbc.M500555200

## Description

### BACKGROUND

The present invention is directed to conjugates and a method for detection of a target moiety in a sample of biological specimens by labeling the target moiety with a conjugate having a peptide/MHC (pMHC) complex targeting TCR and/or KIR molecules expressed on cells and a detection moiety connected via an enzymatically degradable spacer, wherein after detecting or isolating the target moiety, the degradable spacer may be enzymatically degraded, thereby cleaving the detection moieties X from the labeled target moieties and removing detection moieties and optionally pMHC complexes from the target cells.

The identification of antigen-specific T cells is of utmost importance in the field of T cell immunology and T cell therapy. The central event in T cell immunity is the interaction between a peptide/major histocompatibility complex (pMHC) and a T cell receptor, present on CD4+ and CD8+ T cells. T cells are activated upon a specific, usually clonotypic interaction, proliferate and become activated, eventually fulfilling an effector function in the context of cancer defense or anti-viral immunity.

Analysis, detection, identification and isolation of these T cells often requires an oligomeric presentation of T cell receptor (TCR)-cognate pMHCs coupled to a fluorophore-conjugated scaffold. The reason for this requirement is the usual weak monovalent interaction between a pMHC complex and the cognate TCRs (KD values usually in µM-mM range). A multimerization of monovalent molecules to generate multivalent molecules may be applied to overcome this affinity limitation through the avidity effect.

Multimerization of monovalent pMHC by using multimerization scaffolds such as Streptavidin usually relies on non-covalent binding. In other cases, binding of the pMHC complexes to said multimerization scaffolds is covalent, but the multimerization of these scaffolds itself relies on non-covalent interactions. In both cases, this limits the overall stability of pMHC conjugates because an uncontrollable dissociation of conjugates may occur, resulting in potential non-functional conjugates that may have a low specificity for their target molecule.

Besides stable and specific binding of the conjugate to the target moiety, the possibility to cleave the conjugate from the target moiety is of crucial relevance, as multimerized pMHCs might activate T cells when bound to TCRs for a longer period of time. From a clinical point of view, removing MHC-based conjugates from cells, for example before expanding them *ex vivo*, is preferred when these cells should be re-administrated into patients due to safety aspects.

pMHCs can be bound to proteins that mediate multimerization (EP0812331, EP1530592, and EP1377609). Said proteins comprise for instance streptavidin, which itself tetramerizes and therefore presents four biotinylated pMHCs, or other protein-based scaffolds linked to fluorophores forming a pentameric structure. More recent pMHC multimers consist of pMHC complexes conjugated to binding entities such as streptavidin, which themselves are bound to polysaccharide carrier molecules. This leads to an even higher valency.

EP0812331 discloses a multimeric MHC-antigen complex which forms a stable structure with T cells recognizing the complex through their antigen receptor, thereby allowing for the labeling, identification and separation of specific T cells, wherein the complex is multimerized by enzymatical introduction of biotin moieties and subsequent binding of said moieties to a multivalent entity.

EP 1530592 discloses an oligomeric MHC complex comprising at least two chimeric proteins, said chimeric proteins comprising a first section derived from an MHC peptide chain or a functional part thereof and a second section comprising a pentamerisation domain derived from COMP, wherein formation of the oligomeric MHC complex occurs by oligomerisation at the pentamerisation domain of the chimeric proteins.

EP1377609 discloses an MHC molecule construct in soluble form in a solubilizing medium or immobilized onto a solid or semi-solid support, said MHC molecule construct comprising a soluble dextran carrier molecule attached to at least 5 MHC molecules, wherein said MHC molecules are attached to the dextran carrier molecule via more than one binding entity, wherein each binding entity is attached to from 2 to 4 MHC molecules, or wherein said MHC molecules are directly attached to the dextran carrier molecule and said MHC molecule construct comprises one or more labels.

WO2002054065 discloses a method for reversible MHC multimer staining for functional purification of antigen-specific T cells comprising a fusion polypeptide comprising at least one streptavidin-binding peptide and an MHC molecule, wherein the MHC multimer complexes comprising said fusion polypeptides and modified streptavidin molecules are disrupted by adding biotin.

Schmidt et al. (2011 JBC 286, 41723-41735) disclose reversible MHC I-peptide multimers containing Ni2+-NTA peptides and histidine tags for analysis and sorting of CD8+ T cells. Said multimers are dissociated in the presence of imidazole.

Said methods rely on reagents which are multimerized non-covalently, and the reversibility (of binding/staining) is induced by the addition of a competing compound, which itself binds with higher affinity to the multimerization scaffold or is present at higher concentration than the affinity-tagged pMHC molecules. Based on the nature of the interaction, this system is designed to rely on low to moderate affinity of the tag towards the multimerization scaffold. As result, mixing of two multimerization scaffolds each having a different pMHC molecules (i.e. mixing of two reagents) may result in an undesired exchange of pMHC molecules between the two multimerization scaffolds. Such conjugates and staining methods therefore have limited performance with respect to multiplexing potential or bulk / large-scale analyses.

Novel cancer therapy approaches rely on the prediction of cancer-specific mutations and on the specific presentation of peptide fragments, derived from mutated proteins which only occur in certain tumor types, by MHC proteins. In order to identify these so called neoantigens, a cohort of peptides needs to be tested to identify neoantigen-specific T cells displaying anti-tumor activity. This requires a multiplexing approach and preferably pMHC multimers that have a controllable reversible binding to TCRs for the identification and verification of these T cells. Such pMHC multimers, preferably those with a higher stability (thus avoiding exchange of pMHC molecules from different scaffolds with different detection labels) would solve the issue limiting higher throughput identification of T cells while bearing the opportunity for using the analyzed cells afterwards. It would further allow for bulk staining T cells, for example screening for T cells displaying anti-tumor reactivity or anti-neoantigen reactivity.

Regarding the removal of the conjugate from target cells, several approaches for reversible labeling of cells with immunoconjugates are known.

WO 96/31776 discloses a method to release particles from target cells by enzymatically cleaving a moiety of the particle coating, or a moiety present in the linkage group between the coating and the antigen recognizing moiety. An example is the application of magnetic particles coated with dextran and/or linked via dextran to the antigen recognizing moiety. Subsequent cleavage of the isolated target cells from the magnetic particle is initiated by the addition of the dextran-degrading enzyme dextranase.

A similar method is disclosed in EP3037821, with the detection and separation of a target moiety according to, for example a fluorescence signal, with conjugates having an enzymatically degradable spacer for reversible fluorescent labeling.

Conjugates for reversible labeling comprising an enzymatically degradable spacer are further disclosed in PCT/EP2019/060403, wherein the fluorescent quantum yields for detection is increased by inserting a linker unit comprising one or more polyethylene glycol between releasable spacer and fluorescent dye. Therefore, WO 96/31776 is directed to release a magnetic label and EP3037821 and PCT/EP2019/060403 to release a fluorescence signal from a target moiety by enzymatic digestion, but do not disclose a method for covalent conjugation of an antigen recognizing moiety in a directed manner via its C-terminus to ensure a proper oriented presentation and stable binding to the target moiety. There is need in the art for improved pMHC multimers for reversible labeling of cells bearing TCR and/or KIR molecules, especially with respect to multiplexing potential.

Binding and detection of cells via an MHC complex is further descriped in the following patent applications: WO02072631, US2005003431, WO2009039854, WO2010037397, WO2010037395, EP3037821, WO2016198932 and in the following scientific publications:
- O'CALLAGHAN C A ET AL, "BirA enzyme: production and application in the study of membrane receptor-ligand interactions by site-specific biotinylation", ANALYTICAL BIOCHEMISTRY, ACADEMIC PRESS, AMSTERDAM, NL, (19990101), vol. 266, no. 1, doi:10.1006/ABIO.1998.2930, ISSN 0003-2697, pages 9 - 15
- G. DOLTON ET AL, "Comparison of peptide-maj or histocompatibility complex tetramers and dextramers for the identification of antigen-specific T cells", CLINICAL AND EXPERIMENTAL IMMUNOLOGY, GB, (20140609), vol. 177, no. 1, doi:10.1111/cei.12339, ISSN 0009-9104, pages 47 - 63
- LINDA WOOLDRIDGE ET AL, "Interaction between the CD8 Coreceptor and Major Histocompatibility Complex Class I Stabilizes T Cell Receptor-Antigen Complexes at the Cell Surface", JOURNAL OF BIOLOGICAL CHEMISTRY, US, (20050729), vol. 280, no. 30, doi:10.1074/jbc.M500555200, ISSN 0021-9258, pages 27491 - 27501

### SUMMARY

It was therefore an object of the invention to provide a conjugate and a method for specific labeling, subsequent detection, analysis and/or isolation, and de-labeling of cells bearing TCR molecules in a sample of biological specimen in order to enable reversible labeling and further labeling cycles.

Surprisingly, it was found that for a specific and stable binding of pMHC complexes, it is beneficial that the antigen recognizing moiety Y is covalently conjugated via its C-terminus to the further units of the conjugate.

Accordingly, the invention is directed to a conjugate for labelling a target moiety on a cell according to general formula (I)

(I) Xₒ - P - Yₘ

with Y : MHC-complex which is targeting TCR and/or KIR molecules
   P : enzymatically degradable spacer,
   X : fluorescent dye,
   o : integer between 5 and 25,
   m: integer between 2 and 1000
wherein X and P; P and Y are covalently bound to each other **characterized in that** Y is bound to P via the C-terminus of Y.

The conjugates of the invention show an improved staining and detection of cells bearing TCR molecules due to the orientation of Y, i.e. because Y is bound to P via its C-terminus to the spacer P. The prior art is silent on the orientation of pMHC-complexes targeting TCR molecules and its technical effect. For example, the method disclosed in EP3037821 would generate "randomly-oriented" MHC-complexes by conjugating the MHC proteins via their naturally occurring amino acid residues such as cysteine and lysine.

Further object of the invention is a method for detecting a target moiety in a sample of biological specimen by:
a) providing at least one conjugate having the general formula I

   (I) Xₒ - P - Yₘ

   with Y : MHC-complex which is targeting TCR molecules
      P : enzymatically degradable spacer,
      X : fluorescent dye,
      o : integer between 5 and 25,
      m: integer between 2 and 1000
   wherein X and P; P and Y are covalently bound to each other and Y is bound to P via the C-terminus of Y,
b) contacting the sample of biological specimens with the conjugate accoding to formula (I), thereby labeling the target moiety recognized by Y
c) detecting the target moiety labelled with the conjugate with the detection moiety X.

### BRIEF DESCRIPTION OF THE DRAWINGS

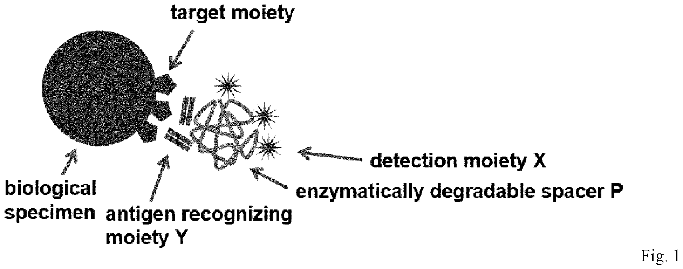

Fig. 1 shows a pMHC molecule construct comprising an enzymatically degradable spacer, wherein the pMHC molecules are "randomly-oriented" due to conjugation via, for example, naturally occurring lysine residues.

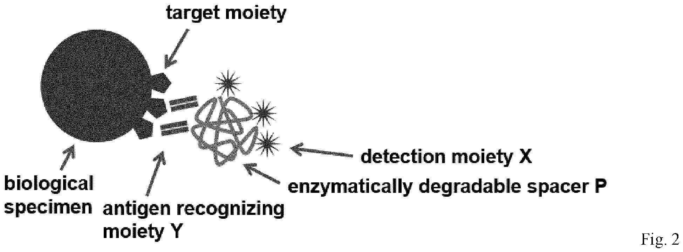

Fig. 2 shows a construct according to general formula I, wherein the pMHC molecules are covalently conjugated via their C-terminus and therefore are presented in an oriented manner.

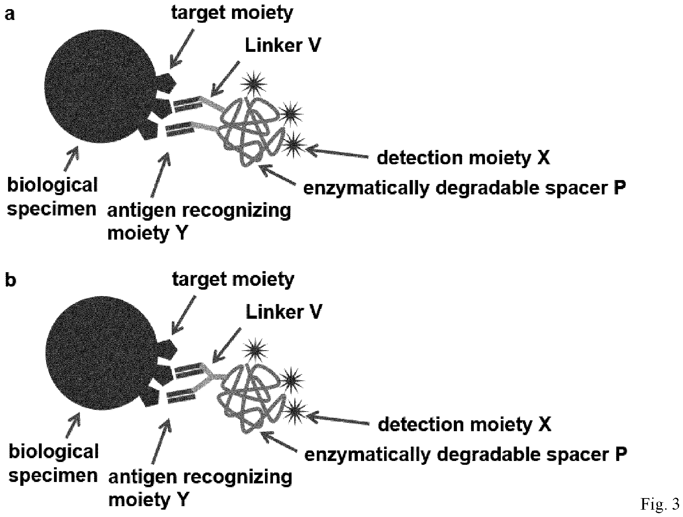

Fig. 3 shows constructs according to general formula III, wherein the pMHC molecules are covalently conjugated via their C-terminus and a linker V in a (a) monovalent or (b) bi-/multivalent manner.

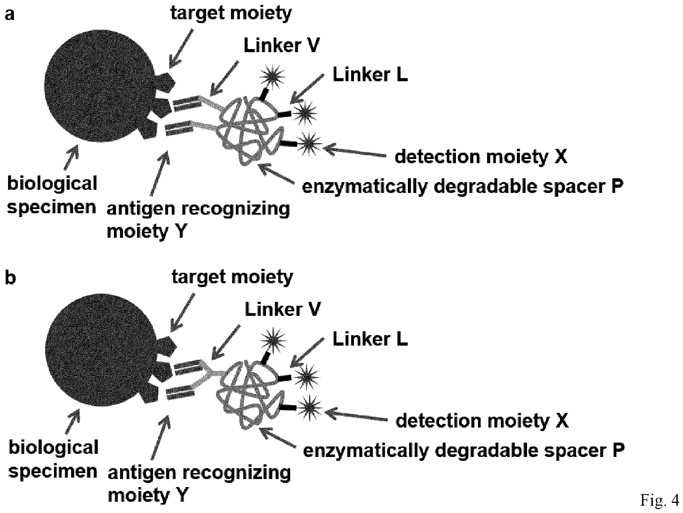

Fig. 4 shows a construct according to general formula IV, wherein the pMHC molecules are covalently conjugated via the C-terminus and a linker V in a (a) monovalent or (b) bi-/multivalent manner, wherein the detection moiety is covalently conjugated to the enzymatically degradable spacer P via the linker L.
Fig. 5 shows the principle of reversible labeling according to the invention with enzymatic degradation of spacer P and thereby (a) cleavage of the detection moiety X from the target moiety or (b) cleavage of the detection moiety X and removal of the antigen recognizing moiety Y from the target moiety.
Fig. 6 shows dot plots of flow cytometry analysis after labeling of cells bearing TCR molecules with a pMHC complex conjugate comprising an enzymatically degradable spacer, wherein the pMHC molecules are conjugated via thiolization of naturally occurring lysine residues.
Fig. 7 shows dot plots of flow cytometry analysis after labeling of cells bearing TCR molecules with a pMHC complex conjugate according to general formula I, wherein the pMHC molecule is covalently conjugated via its C-terminus.
Fig. 8 shows dot plots of flow cytometry analysis after labeling of cells bearing TCR molecules with a pMHC complex conjugate according to general formula III, wherein the pMHC molecule is covalently conjugated via its C-terminus and a linker V in bi-/multivalent manner, and the spacer P is enzymatically degraded.
Fig. 9 shows dot plots of flow cytometry analysis after labeling of cells bearing TCR molecules and a subsequent cleavage of pMHC complex conjugates according to general formula III, wherein the pMHC molecule is covalently conjugated via its C-terminus and a linker V in bi-/multivalent manner, wherein the detection moiety is covalently conjugated to the enzymatically degradable spacer P via the linker L.

### DETAILED DESCRIPTION

The method and the conjugate of the invention are preferably used for a specific *in vitro* detection of target cells, but also may be used for a specific *in vitro* isolation of target cells.

The units X, P, and Y of the conjugate as claimed may be bound to each other via one or more linkers, which is subject of certain embodiments of the invention. Without being bound to this theory, it is believed that such linkers increase the distance between the moieties and/or the degree of multimerization and accordingly the quantum yield of the conjugate.

In a first embodiment of the invention, the conjugate according to the invention is characterized in that X is bound to P via linker L according to general formula (II) (Xₒ-L)ₙ - P - Yₘ with L: linker unit, n : integer between 1 and 1000 and the proviso that o * n <= 1000.

In a second embodiment of the invention, the conjugate according to the invention is characterized in that Y is bound to P via linker V according to general formula (III) Xₒ - P - (V- Yₘ)_{q} with V : linker unit, q : integer between 2 and 1000 and with the proviso that q * m <= 1000.

In a third embodiment of the invention, the conjugate according to the invention is characterized in that X is bound to P via linker L and Y is bound to P via linker V according to general formula (IV) (Xₒ-L)ₙ - P - (V- Yₘ)_{q} with V, L: same or different linker unit, q : integer between 2 and 1000, n : integer between 1 and 1000 and with the provisos that o * n <= 1000 and q * m <= 1000.

In a fourth embodiment, any combination of the embodiments described in formula (I) to (IV) is encompassed. In this embodiment of the invention, the conjugate according to the invention is characterized in that X is bound to P via linker L and Y is bound to P via linker V according to general formula (IV) (Xₒ-L)ₙ - P - (V- Yₘ)_{q} with V, L: same or different linker unit, q : integer between 2 and 1000, n : integer between 1 and 1000 and with the provisos that o * n <= 1000 and q * m <= 1000.

### Target moiety

The target moiety to be detected with the method of the invention can be in and/or on any biological specimen, such as for example whole animals, physiological samples, organs, tissues, cells, single cells, cell aggregates, organoids, suspension cells, (e.g. PBMCs or cell derived from lysed tumor), *in vitro* cultured cells, adherent cells, an extracellular matrix or matrices, extracellular vesicle(s), organelle(s), whole blood, plasma, urine, feces, saliva, viral particles, or a combination thereof.

Said biological specimen may be alive or dead. Preferably, target moieties are molecules being present on and/or in said biological specimen specifically recognizing peptide-bound MHC molecules or ligand-bound MHC-like molecules (such as CD1(a-e) and MHC-related protein 1 (MR1)) of non-mammalian vertebrates (such as salmonids and chicken) and of mammalian vertebrates (such as mice and humans). More preferred, said target moieties are proteins on the surface of cells such as receptors. Said proteins may be antigens. In particular, target cells may be antigen-specific T cells carrying a T cell receptor (TCR). Said antigen-specific T cells may be CD4+ T cells, expressing a CD4 coreceptor binding to MHC class II proteins and/or CD8+ T cells expressing the CD8 coreceptor binding to MHC class I proteins. Target cells may also comprise NK cells carrying a killer cell immunoglobulin-like receptor (KIR) known to interact with MHC complexes.

The target moiety may be present on cells selected from, for example, CD3+ T cell, CD4+ T cells, CD8+ T cells, T helper cells, cytotoxic T cells, alpha beta T cells, gamma delta T cells, NK cells, NKT cells, and cells expressing recombinant TCR and/or KIR molecules.

Said target cells representing said target moieties may be antigen-specific and/or pathogen-reactive lymphocytes (for example virus-reactive lymphocytes, bacteria-reactive lymphocytes, and lymphocytes showing an antigen-specific reactivity against other types of parasites), autoimmune-reactive lymphocytes, tumor-infiltrating lymphocytes (TILs), tumor-reactive lymphocytes (TLRs), and lymphocytes showing allo- or xeno-reactivity.

The target cell may be a T cell expressing a TCR recognizing a cancer-related antigenic peptide and/or a neoantigenic peptide presented by a pMHC molecule, or a TCR recognizing either an autoimmune-relevant peptide or a pathogen-derived antigenic peptide bound to an MHC molecule.

Target cells may be genetically modified expressing exogenous TCR and/or KIR, wherein said TCR and/or KIR may be genetically engineered and optimized for pMHC recognition (for example showing a higher affinity towards a pMHC complex compared to its wildtype analog).

The target moiety may be a fragment of a TCR or a KIR. Examples comprise single-chain TCR (fusion of Vα and Vβ part of the TCR) and chimeric TCR fusion protein comprising at least a fragment of the TCR fused to another protein such as a scFv or a nanobody).

The target cell may express antibodies, antibody-derived fragments, or molecules with antibody-like properties (antibody mimetics) recognizing pMHC molecules or ligand-bound MHC-like molecules. Antibody-derived fragments and molecules with antibody-like properties comprise, for example, Fab, Fab', F(ab')2, sdAb, scFv, di-scFv, scFv-Fc, nanobodies, DARPins, Anticalins, and monobodies.

### Antigen recognizing moiety Y

The term "antigen recognizing moiety Y" refers to any kind of pMHC or MHC-like molecule which specifically binds a target moiety expressed in and/or on biological specimens.

The term "antigen recognizing moiety Y" relates especially to pMHC- or MHC-like complexes targeting, for example, TCRs, KIRs, and other pMHC-recognizing receptors. The antigen-recognizing moiety may be an initially peptide-free MHC complex being peptide-receptive which is able to bind an antigenic peptide prior to the detection, analysis and/or isolation of the target moiety present on the target cell. The antigen-recognizing moiety may be an MHC molecule bound to a placeholder peptide or peptide fragment, wherein said peptide or peptide fragment can be replaced by another antigenic peptide upon the addition of said antigenic peptide, for example an infectious disease or cancer-related peptide derived from a protein antigen. The antigen-recognizing moiety may further be an MHC-like molecule bound to a lipid or a small molecule such as a vitamin or metabolite.

The term "specific" with respect to the binding of an antigen recognizing moiety to a target moiety and the labeling of a target moiety by a detection moiety refers to a specific interaction, either direct or indirect, between two molecules, for example the specific binding of a pMHC conjugate to an antigen (TCR) on a target cells, wherein said pMHC does not substantially recognize or bind other antigens (TCRs) on said target cell, and the labeling of said target cell by said conjugate. An antigen recognizing moiety that binds specifically to an antigen from one species may bind also to that antigen from another species.

The conjugate used in the method of the invention may comprise 2 to 1,000, preferably 2 to 100, more preferred 2 to 50, more preferred 2 to 40, and most preferred 2 to 30 antigen recognizing moieties Y. These preferred ranges correspond with appropriate ranges of m and o according to the embodiments as already disclosed. For example, if m equals 20, q in general formula (IV) may be at most 50 to fulfill the proviso q * m <= 1000.

The interaction of the antigen recognizing moiety with the target moiety may be of high or low affinity. Binding interactions of a single low-affinity antigen recognizing moiety are too low to provide a stable binding to the antigen. Low-affinity antigen recognizing moieties may be multimerized by, for example, conjugation of said moieties to an enzymatically degradable spacer P to create and/or increase avidity.

Preferably, the term "antigen recognizing moiety Y" refers to peptide-bound or peptide-receptive classical major histocompatibility complex (MHC) class I and MHC class II and non-classical MHC molecules, as well as MHC-like molecules bound to, for example, lipids and small molecules such as vitamins or metabolites. The "Antigen recognizing moiety Y" comprises classical and non-classical human (HLA), ovine (OLA), bovine (BoLA), rat (RT1), murine (H-2), equine (ELA), non-human primates (NHP), and swine (SLA) MHC complexes. Peptide-receptive MHC complexes may refer to stabilized, peptide-free MHC proteins (for example by the introduction of 2 cysteine residues within the peptide binding groove to stabilize said peptide-free MHC) and to peptide-receptive MHC proteins resulting from the occupation of the peptide binding groove by short peptide fragments (<8 amino acids) or low-affinity peptides. In case of human MHC complexes, the "Antigen recognizing moiety Y" comprises human HLA-A, HLA-B, HLA-C, HLA-DR, HLA-DQ, HLA-DP, HLA-E, HLA-E, HLA-G, MR1, and CD1(a-e) complexes.

In case the "antigen recognizing moiety Y" is an MHC class I molecule, it preferably may include the extracellular domain of the MHCI heavy chain (α chain) bound to the beta-2-microglobulin (β2m) domain and an antigenic peptide.

The "antigen recognizing moiety Y" may also refer to a molecule comprising the full length MHCI heavy chain (α chain) bound to the beta-2-microglobulin (β2m) domain and an antigenic peptide.

Furthermore, the "antigen recognizing moiety Y" may also refer to a molecule comprising the full length MHCI heavy chain (α chain) bound to the beta-2-microglobulin (β2m) domain and an antigenic peptide, wherein said peptide is fused to either the α chain or the β2m domain.

Other variants of the "antigen recognizing moiety Y" may include, for example, the TCR-binding relevant parts of the MHC complexes, for example single-chain MHC constructs comprising the α1- α2 domains fused to each other and bound to a peptide, wherein said peptide optionally may be fused to either the α1 or α2 domain or the β2m domain.

The antigen recognizing moiety Y may refer to a fragmented MHC molecule complex.

One or several parts (chains or fragmented domains) of the pMHC complex or parts of MHC-like molecule may be fused to biologically active entities, itself displaying immune-modulating function. Said biologically active entities may comprise cytokines, growth factors, and hormones. Alternatively, said parts (chains or fragmented domains) of the pMHC complex or parts of MHC-like molecule may be fused to interacting entities such as antibodies, antibody-derived fragments, molecules with antibody-like properties comprise, and peptides.

The "antigen recognizing moiety Y" may comprise α and β chains bound to a peptide, wherein both α and β chains may be dimerized by, for example, fusion to leucine zipper domains. The peptide occupying the peptide binding groove may be genetically tethered to the N-terminus of the α and/or β chain via a linker and the linker may be enzymatically cleavable, for example by a protease.

The antigen recognizing moiety Y may be covalently coupled to the spacer P via its C-terminus. Methods for covalent conjugation are known by persons skilled in the art and are described in detail in the section about the linker V. When the antigen recognizing moiety, Y comprises more than one C-terminus, each C-terminus may be used for coupling.

For proteins, the term "C-terminus" as used herein comprises the C-terminal region of a protein, preferably the region with the last 30 amino acids, more preferred the region with the last 20 amino acids, more preferred the region with the last 10 amino acids, more preferred the region with the last 5 amino acids, more preferred the region with the last 3 amino acids, more preferred the region with the last 2 amino acids, and most preferred the region with the last amino acid (the C-terminal amino acid).

### Detection moiety

In the broader disclosure, the detection moiety X of the conjugate may comprise any moiety possessing a property or function that can be used for detection, analysis, and/or isolation of cells. Preferably, the detection moiety X is selected from the group comprising chromophore moiety, fluorescent moiety, phosphorescent moiety, luminescent moiety, light absorbing moiety, radioactive moiety, transition metal and isotope mass tag moiety, magnetic moiety/particle, solid support with shape of particles (for example sheets, plates, membranes, tubes, columns, wells, or micro-arrays).

In the invention as claimed however, the detection moiety X is a fluorescent moiety. Suitable fluorescent moieties are those known from the art of immunofluorescence technologies, for example flow cytometry or fluorescence microscopy. The target moiety may be labeled with the conjugate and may be detected by exciting the detection moiety X and detecting the resulting emission (photoluminescence).

Useful fluorescent moieties may be small organic molecule dyes, such as xanthene dyes, fluorescein, rhodamine dyes, coumarin dyes, cyanine dyes, pyrene dyes, oxazine dyes, pyridyl oxazole dyes, pyromethene dyes, acridine dyes, oxadiazole dyes, carbopyronine dyes, benzpyrylium dyes, fluorene dyes, or metallo-organic complexes, such as Ru, Eu, Pt complexes. Besides single molecule entities, clusters of small organic molecule dyes, fluorescent oligomers or fluorescent polymers, such as polyfluorene, may also be used as fluorescent moieties. Additionally, fluorescent moieties may be protein-based, such as phycobiliproteins, nanoparticles, such as quantum dots, upconverting nanoparticles, gold nanoparticles, and dyed polymer nanoparticles. The fluorescent moiety may comprise at least two covalently attached fluorescent molecules. The excitation and emission properties of such fluorescent dyes are based on fluorescence resonance energy transfer.

Photoluminescent detection moieties may also comprise phosphorescent moieties with time-delayed emission of light after excitation. Phosphorescent moieties may include metallo-organic complexes, such as Pd, Pt, Tb, Eu complexes, or nanoparticles with incorporated phosphorescent pigments such as lanthanide doped SrAl₂O₄.

The target moiety labeled with the conjugate may be detected without prior excitation by irradiation, for example using a radioactive label as detection moiety. The labeling may be conducted by exchanging non-radioactive isotopes for their radioactive counterparts, such as tritium, 32P, 35S or 14C, or by introducing covalently bound labels, such as 125I, which is bound to tyrosine, 18F within fluorodeoxyglucose, or metallo-organic complexes, i.e. 99Tc-DTPA.

The detection moiety may can cause chemiluminescence, for example a horseradish peroxidase label in the presence of luminol.

The target moiety labeled with the conjugate may be detected by light absorption, preferably UV light, visible light, or NIR light. Suitable light-absorbing detection moieties comprise light absorbing dyes without fluorescence emission, such as small organic molecule quencher dyes like N-aryl rhodamines, azo dyes, and stilbenes. The light-absorbing detection moiety may be irradiated by pulsed laser light, generating a photoacoustic signal.

The target moiety labeled with the conjugate may also be detected by mass spectrometric detection of a transition metal isotope. Transition metal isotope mass tag labels may be introduced as covalently bound metallo-organic complexes or nanoparticle component. Isotope tags of lanthanides and adjacent late transition elements are known in the art.

Furthermore, the detection moiety may be a solid support possessing a property or function which can be used for detection, analysis and isolation of cells. Suitable solid supports are known in biotechnology for immobilizing cells and may have the shape of particles, for example, sheets, plates, membranes, tubes, columns, wells, or micro-arrays manufactured from various materials such as polystyrene (PS), polymethylmethacrylate (PMMA), polyvinyl toluene (PVT), polyethylene (PE), or polypropylene (PP). Suitable materials are commercially available.

The solid support may be further a magnetic particle, also known in the art as nano- to microscale magnetic bead. The mean diameter of the beads may range from 10 nm to 10 µm. Biocompatible magnetic particles are commercially available and comprise, for example, forms of magnetically iron oxide coated by a shell of dextran molecules or silica. The solid support may also be polymers containing magnetic materials. Suitable particles are commercially available from Miltenyi Biotec B.V. & Co. KG, Germany under the trade name "MicroBeads" and "MACSiBeads" possessing a hydrodynamic diameter of 50-100 nm or 3-4 µm, respectively.

The conjugate used in the method of the invention comprises between 5 and 25 detection moieties X. These preferred ranges correspond with appropriate ranges of m and o according to the embodiments as already disclosed. For example, if n equals 50, o in general formula (IV) may be at most 20 to fulfill the proviso o * n <= 1000.

The antigen detection moiety X may be covalently coupled to the spacer P. Methods for covalent conjugation are known by persons skilled in the art and are described in detail in the section about the linker L.

The detection moiety X may be conjugated via a chemical reaction of an activated group either to a second detection moiety X (tandem detection moiety) or to the spacer P, wherein a functional group may be present on either the spacer P or on the second detection moiety X. The second detection moiety may be identical to or different from the first one.

### Enzymatically degradable spacer P

The enzymatically degradable spacer P may be any molecule which can be cleaved by an enzyme. Preferable enzymatically degradable spacer P is selected from the group consisting of polysaccharides, proteins, peptides, depsipeptides, polyesters, nucleic acids, and derivatives thereof.

It is beneficial for the staining step that the enzymatically degradable spacer P has a certain size in order to provide steric space for the detection moieties X. On the other hand, a enzymatically degradable spacer P which too large takes more time to digest by the enzyme, resulting in loger process time or worse, in an incomplete release. It has been forund that the size of the enzymatically degradable spacer P and the respective average amounts of detection moieties X and MHC-complex targeting TCR and/or KIR molecule Y has an effect on staining and destaing (release).

In preferred embodiments of the invention, the conjugates are provided with enzymatically degradable spacers P having a size as defined by molecular weight of 50-150 kDa, 150 to 300 kDa or 300 to 700 kDa.

The index "o" in general formulas (I) to (IV) refers to the degree of labeling of pMHC per spacer P, preferable to the degree of labeling of pMHC per dextran-fluorescein as follows:

For enzymatically degradable spacers P having a size as defined by molecular weight of 50-150 kDa, the average number of detection moieties X per P is preferable between 1 and 4, more preferable between 1 and 2. Independently, the average number of MHC-complex targeting TCR and/or KIR molecule Y per P is preferable between 5 and 10, more preferable between 2 and 5. In the invention furthermore the requirements of the independent claims must also be met.

For enzymatically degradable spacers P having a size as defined by molecular weight of 150 to 300 kDa, the average number of detetion moitoies X per P is preferable between 1 and 5, more preferable between 1 and 3. Independently, the average number of MHC-complex targeting TCR and/or KIR molecule Y per P is preferable between 6 and 15, more preferable between 6 and 10. In the invention furthermore the requirements of the independent claims must also be met.

For enzymatically degradable spacers P having a size as defined by molecular weight of 300 to 700 kDa, the average number of detetion moitoies X per P is preferable between 1 and 6, more preferable between 1 and 3. Independently, the average number of MHC-complex targeting TCR and/or KIR molecule Y per P is preferable between 10 and 20, more preferable between 10 and 15. In the invention furthermore the requirements of the independent claims must also be met.

The spacer P may be covalently conjugated to the antigen recognizing moiety Y, optionally via the linker V and/or L and to the detection moiety X. In the invention furthermore the requirements of the independent claims must also be met.

Suitable polysaccharides comprise, for example, dextrans, pullulans, inulins, amylose, cellulose, hemicelluloses such as xylan or glucomannan, pectin, chitosan, and chitin, which may be derivatized to provide functional groups for covalent binding of the linker V, the antigen recognizing moiety Y, the linker L and the detection moiety X.

Suitable enzymes comprise, for example, glycosidases, dextranases, pullulanases, amylases, inulinases, cellulases, hemicellulases, pectinases, chitosanases, chitinases, proteinases, peptidases, hydrolases, esterases, lipases, and nucleases.

The enzymatically degradable spacer P may be modified. A variety of such modifications are known in the art, for example, imidazolyl carbamate groups may be introduced by reacting the polysaccharide with N,N'-carbonyl diimidazole. Subsequently amino groups may be introduced by reacting said imidazolyl carbamate groups with hexane diamine. Polysaccharides may be oxidized using periodate to provide aldehyde groups or with N,N'-dicyclohexylcarbodiimide and dimethyl sulfoxide to provide ketone groups. Aldehyde or ketone functional groups may be reacted subsequently preferably under conditions of reductive amination either with diamines to provide amino groups or directly with amino substituents on a proteinaceous binding moiety. Carboxymethyl groups may be introduced by treating the polysaccharide with chloroacetic acid. Activating the carboxy groups with methods known in the art which yield activated esters such N-hydroxysuccinimid ester or tetrafluorophenyl ester allows for reaction with amino groups either of a diamine to provide amino groups or directly with an amino group of a proteinaceous binding moiety. It is generally possible to introduce functional group bearing alkyl groups by treating polysaccharides with halogen compounds under alkaline conditions. For example, allyl groups may be introduced by using allyl bromide. Allyl groups may be further used in a thiol-ene reaction with thiol bearing compounds such as cysteamine to introduce amino groups or directly with a proteinaceous binding moiety with thiol groups generated by reduction of disulfide bonds or introduced by thiolation for instance with 2-iminothiolane.

Proteins, peptides, and depsipeptides used as enzymatically degradable spacer P may be functionalized via side chain functional groups of amino acids to attach the spacer P to linker L and to the antigen recognizing moiety Y. Side chains functional groups suitable for modification may be for instance amino groups provided by lysine, or thiol groups provided by cysteine after reduction of disulfide bridges.

Polyesters and polyester amides used as enzymatically degradable spacer P may be synthesized with co-monomers that provide side chain functionality, or they may be subsequently functionalized.

In the case of branched polyesters functionalization may done via the carboxyl or hydroxyl end groups. Post polymerization functionalization of the polymer chain may be done, for example, via addition to unsaturated bonds, i.e. thiolene reactions or azide-alkine reactions, or via introduction of functional groups by radical reactions.

Nucleic acids used as enzymatically degradable spacer P may be preferably synthesized with functional groups at the 3' and 5' termini suitable for attachment of the binding moiety B and antigen recognizing moiety A. Suitable phosphoramidite building blocks for nucleic acid synthesis providing for instance amino or thiol functionalities are known in the art.

The enzymatically degradable spacer P may be composed of more than one different enzymatically degradable units, which are degradable by the same or by different enzymes.

### Linker units L and V

As already described in further instances of the disclosure, the detection moiety X and/or the antigen recognizing moiety Y may be covalently coupled to the spacer P via linker units L and/or V. Methods for covalent conjugation involving such linkers are known by a person skilled in the art and are described later. In the invention furthermore the requirements of the independent claims must also be met.

In general, linker units L and V may be any polar hydrophilic oligomer, comprising between 1 and 1,000, preferably between 1 and 500, more preferred between 2-100, and most preferred between 2-50 carbon and hetero atoms.

Preferable, linker units V and L are selected from the group consisting of polyethylene glycol, peptides, proteins, polysaccharides, depsipeptides, polyesters, nucleic acids, polyvinylpyrrolidones, polyacrylates, polymethacrylates, polyoxazolines, polyvinyl alcohols, polyacrylamides, polycarbonates and derivatives or mixtures thereof. V and L may be same or different.

The linker units L and/or V may be linear to allow for the attachment of a single detection moiety X and/or antigen recognizing moiety Y. The linker unit may comprise a functional or activated group on each end to react directly or via prior reaction with a heterobifunctional cross linker with an activated or functional group on the detection and/or antigen recognizing moieties X and Y and with an activated or functional group on the enzymatically degradable spacer P. Alternatively, the detection moiety X and/or antigen recognizing moiety Y may comprise a group like an polyethylene glycol chain with an activated or functional group, which can then be conjugated to the enzymatically degradable spacer P.

Commercially available heterobifunctional polyethylene glycols may be reacted with an activated detection moiety X and/or antigen recognizing moiety Y on one end and can be activated on the other end for reaction with the enzymatically degradable spacer P.

The linker units L and/or V may also be branched to allow for the attachment of multiple detection moieties X and/or antigen recognizing moieties Y, wherein the polyethylene glycol is bound to at least one (preferably one to ten) polyhydroxy branching units chosen from core unit selected from the group comprising polyhydroxy compounds, polyamino compounds, and polythio compounds.

Commercially available multi-arm polyethylene glycols (branched PEGs) may serve as linkers which include a branching moiety and polyether branches. The ends of the arms of the branched PEGs may provide functional or activated groups.

Preferred as core unit for multi-arm polyethylene glycols (branched PEGs) are, for example, glycerol with three hydroxyl groups as attachment point for 3 polyether residues via ether bonds, pentaerythritol with four hydroxyl group as attachment points for 3 to 4 polyether residues via ether bonds, dipentaerythritol with six hydroxyl groups as attachment points for 3 to 6 polyether branches via ether bonds, tripentaerythritol or hexaglycerol with eight hydroxyl groups as attachment points for 3 to 8 polyether branches via ether bonds. Commercially available examples comprise MAL-PEG(12)-[PEG(11)-MAL]3, and Tetra(-amido-PEG-MAL)pentaerythritol. Such branched linker units L and/or V comprise a sum of 1 to 1,000 ethylene glycol repeating units.

Other multivalent linker comprise amino acids, e.g. lysine, histidine, tryptophan, arginine, tyrosine, serine, threonine, glutamic acid, asparagine, methionine, and cysteine, with 3 attachment points. An illustrative example is the commercially available MAL-L-Lys(MAL)-PEG4-TFP.

The detection moieties X and/or antigen recognizing moieties Y may be conjugated via an enzymatic reaction to the spacer P. The motif or tags required for enzymatic ligation may be part of the detection moiety X and/or antigen recognizing moiety Y. In addition, the motif or tags may be introduced by any chemical or enzymatic reaction to the detection moiety X and/or antigen recognizing moiety Y.

In addition, the detection moieties X and/or antigen recognizing moieties Y may be conjugated via a homobifunctional linker molecule providing the same activated/functional group or motif/tag or a heterobifunctional linker molecule, which is firstly chemically or enzymatically reacted with one and secondly with the other activated/functional group or motif/tag.

### Coupling chemistry

The detection moieties X and/or antigen recognizing moieties Y are covalently coupled to spacer P, optional via linker units V and/or L. Methods for such covalent conjugation are known by the person skilled in the art and are exemplary described.

For the conjugation of MHC I and MHC II molecules the C-terminus of α- and/or β-chain may be used. For MHC I and MHC-like molecules, the covalent conjugation via the C-terminus of the α-chain (heavy chain) is preferred, while for MHC II molecules the conjugation to the C-termini of both chains (α- and/or β-chain) are suitable.

The detection moieties X and/or antigen recognizing moieties Y may be conjugated via a chemical reaction of an activated group either on the detection moieties X and/or antigen recognizing moieties Y or on the spacer P with an appropriate functional group on either the spacer P or on the antigen recognizing moiety Y. In case the detection moieties X and/or antigen recognizing moieties Y are bound via linker units V and/or L, the linker units V and/or L are provided with an activated or functional group.

Examples of activated and functional groups comprises amino, thiol, thioether, hydroxyl, phenol, carboxyl, active ester (for instance N-hydroxysuccinimide ester (NHS), sulfodichlorophenyl ester (SDP), tetrafluorophenyl ester (TFP), and pentafluorophenyl ester (PFP)), isothiocyanate, cyanate, sulfonyl chloride, aminooxy, hydrazide, hydrazon, phosphate, maleimide, haloacetamide, iodoacetamide, halides, bromomaleimide, pyridyl disulfide, aldehyde, ketone, boronic acid, azide, alkene, alkyne, cyclooctyne, DIBO, nitrile, cyclopentadiene, tetrazine, phosphine, phosphite, thioester.

Chemical reactions between functional groups and reaction products comprise, for example: amide (e.g. between an amino and active ester or between amino and carboxyl via activation with 1-ethyl-3-(-3-dimethylaminopropyl) carbodiimide hydrochloride (EDC HCl) and NHS or sulfo-NHS or with N', N'-dicyclohexyl carbodiimide (DCC)), imine (e.g. between aldehyde and primary amino), reductive amination (e.g. of imine to secondary amine), hydrazon or oxime bond (e.g. between aldehyde or ketone and hydrazide or aminooxy), thiourea bond (e.g. between an amino and isothiocyanate), thioether bond (e.g. via Michael-Addition between thiol and maleimide, or between thiol and iodoacetamide), disulfide (e.g. via disulfide exchange between thiol and pyridyl disulfide), ester (e.g. between hydroxyl and active ester), click reaction/cycloaddition (e.g. copper(I)-catalyzed alkyne-azide cycloaddition (CuAAC) between azide and alkyne or Strain-promoted azide-alkyne cycloaddition (SPAAC) between azide and cyclooctyne or Diels-Alder reaction between maleimide and cyclopentadiene or (inverse) Diels-Alder reaction between alkene and tetrazine), Staudinger reaction and Staudinger ligation (e.g. between azide and phosphine), Staudinger phosphite (e.g. between azide and phosphite), thiol-ene (e.g. between alkene and thiol), native chemical ligation (e.g. between C-terminal thioester and N-terminal cysteine).

Said activated or functional groups may be fused to the protein sequence of the MHC at its C-terminus. Various amino acids and peptides may be introduced and/or added to the sequence as linker. Natural amino acids with functional side chains comprise, for example, lysine, histidine, tryptophan, arginine, tyrosine, serine, threonine, glutamic acid, glutamine, aspartic acid, asparagine, threonine, methionine, and cysteine.

Single cysteine residues added to the α- and/or β-chain of the C-terminus may be oxidized to disulfides with for example a mixture of oxidized and reduced glutathione during folding of the MHC-complexes. Said disulfides may be reduced to thiols by the addition of reduction agents such as β-mercapto-ethanol, dithiothreitol (DTT), Tris(hydroxymethyl)phosphine (THP), and Tris(2-carboxyethyl)phosphin (TCEP). Non-natural amino acids may provide non-proteinaceous functional groups such as azide, alkene, alkyne, and aldehyde.

In addition, functional or activated groups may be introduced by any chemical or enzymatic reaction to the C-terminal sequence. Illustrative examples for chemical conversion of functional groups comprise 2-iminothiolane converting an amino to a thiol group and succinic anhydride converting an amino to a carboxylic acid. Examples for enzymatic conversion comprise the formation of an aldehyde from a cysteine in the motif CxPxR via formylglycine-generating enzyme (FGE) and the formation of lipoate derivatives in lysine side chain within β-hairpin turns of proteins by lipoic acid ligase (LplA).

The detection moieties X and/or antigen recognizing moieties Y may be conjugated via an enzymatic reaction to the spacer P (or linker units L and/or L). Suitable enzymes comprise ligases that catalyze the formation of a covalent bond between molecule. Example comprise peroxidase that crosslinks tyrosine residues, sortase that cleaves specifically the carboxyl end of threonine in a C-terminal sequence containing an LPxTG motif and then connects the threonine to the amino group of for example an N-terminal glycine residue through a peptide bond, and transglutaminase that catalyzes the reaction between a glutamine side chain and a primary amino group, for example in lysine, via an acyl transfer reaction.

The motif or tags required for enzymatic ligation may be part of the sequence of the MHC at its C-terminus. Various amino acids and peptides may be introduced and added to the sequences as linker. Said motifs and tags may be used in combination. In addition, the motif or tags may be introduced by any chemical or enzymatic reaction to the C-terminal sequence.

In addition, the antigen recognizing moiety may be conjugated via a homobifunctional linker molecule providing the same activated/functional group or motif/tag or heterobifunctional linker molecule, which is firstly chemically or enzymatically reacted with one and secondly with the other activated/functional group or motif/tag.

For example, many heterobifunctional compounds are available for linking to entities. Illustrative entities include: azido benzoyl hydrazide, N-[4-(p-azidosalicylamino)butyl]-3'-[2'-pyridyldithio]propionamide), bis-sulfosuccinimidyl suberate, dimethyl adipimidate, disuccinimidyl tartrate, N-y-maleimidobutyryloxysuccinimide ester, N-hydroxy sulfosuccinimidyl-4-azido benzoate, N-succinimidyl [4-azidophenyl]-1,3'-dithio propionate, N-succinimidyl [4-iodoacetyl]amino benzoate, glutaraldehyde, succinimidyl-[(N-maleimidopropionamido) polyethyleneglycol] esters (NHS-PEG-MAL), and succinimidyl 4-[N-maleimidomethyl]cyclohexane-1-carboxylate. Preferred linking groups comprise 3-(2-pyridyldithio)propionic acid N-hydroxysuccinimide ester (SPDP) and 4-(N-maleimidomethyl)-cyclohexane-1-carboxylic acid N-hydroxysuccinimide ester (SMCC) with a reactive sulfhydryl group on the detection moieties X and/or antigen recognizing moieties Y and a reactive amino group on the spacer P (or the linker units V and/or L).

### Enzymes for degrading spacer P

The enzymatically degradable spacer P is degraded by the addition of an appropriate enzyme. The choice of enzyme as cleavage reagent (release reagent) is determined by the chemical nature of the enzymatically degradable spacer P and can be one enzyme or a mixture of different enzymes.

Enzymes are preferably hydrolases, but lyases or reductases are also possible. Preferable enzymes may be selected from the group comprising glycosidases, dextranases, pullulanases, amylases, inulinases, cellulases, hemicellulases, pectinases, chitosanases, chitinases, proteinases, peptidases, esterases, lipases, and nucleases.

For example, if the spacer P is a polysaccharide, glycosidases (EC 3.2.1) may be most suitable to degrade the spacer P. Preferred are glycosidases that recognize specific glycosidic structures, e.g., dextranase (EC3.2.1.11), which cleaves at the α(1->6) linkage of dextrans; pullulanases, which cleave either α (1->6) linkages (EC 3.2.1.142) or α (1->6) and α (1->4) linkages (EC 3.2.1.41) of pullulans; neopullulanase (EC 3.2.1.135) and isopullulanase (EC 3.2.1.57), which cleave α (1->4) linkages in pullulans; α-Amylase (EC 3.2.1.1) and maltogenic amylase (EC 3.2.1.133), which cleave α(1->4) linkages in amylose; inulinase (EC 3.2.1.7), which cleaves β(2->1) fructosidic linkages in inulin; cellulase (EC 3.2.1.4), which cleaves at the β(1->4) linkage of cellulose; xylanase (EC 3.2.1.8), which cleaves at the β(1->4) linkages of xylan; pectinases such as endo-pectin lyase (EC 4.2.2.10), which cleaves eliminative at the α (1->4) D-galacturonan methyl ester linkages; or polygalacturonase (EC 3.2.1.15), which cleaves at the α(1->4) D-galactosiduronic linkages of pectin; chitosanase (EC 3.2.1.132), which cleaves at the β(1->4) linkages of chitosan; and endo-chitinase (EC 3.2.1.14) for cleaving of chitin.

Proteins and peptides may be cleaved by proteinases, which may be sequence specific to avoid degradation of target proteins on cells. Sequence specific proteases comprise, for instance, TEV protease (EC 3.4.22.44), which is a cysteine protease cleaving at the sequence ENLYFQ\(G or S), enteropeptidase (EC 3.4.21.9), which is a serine protease cleaving after the sequence DDDDK, factor Xa (EC 3.4.21.6), which is a serine endopeptidase cleaving after the sequences IEGR or IDGR, or HRV3C protease (EC3.4.22.28), which is a cysteine protease cleaving at the sequence LEVLFQ\GP.

Depsipeptides, which are peptides containing ester bonds in the peptide backbone, or polyesters may be cleaved by esterases, such as porcine liver esterase (EC 3.1.1.1) or porcine pancreatic lipase (EC 3.1.1.3). Nucleic acids may be cleaved by nucleases, which may be sequence specific, such as restriction enzymes (EC 3.1.21.3, EC 3.1.21.4, EC 3.1.21.5), such as *Eco*RI, *Hind*III, or *Bam*HI, or which may degrade nucleic acids in a non-specific manner, such as DNAse I (EC 3.1.21.1) or Benzonase^{®} endonuclease.

The amount of enzyme added needs to be sufficient to substantially degrade the spacer in the desired time period. Usually the efficiency (i.e. the percentage of spacer cleaved by an enzyme) is at least about 80%, preferably at least about 85%, more preferred at least about 90%, more preferred at least about 95%, and most preferred at least about 99%.

It is not necessary to degrade spacer P entirely or completely. For the method of the invention, it is necessary to degrade spacer P as much that the detection moiety X or the detection moiety X and antigen recognizing moiety Y can be removed from the labeled target moiety by dissociation and/or washing.

The reaction conditions for degradation may be empirically optimized in terms of, for example, temperature, pH, presence of metal cofactors, reducing agents. The substantial degradation of spacer P will usually be completed in less than about 2 hours, preferably in less than about 1 hour, more preferred in less than about 30 minutes, more preferred in less than about 15 minutes, and most preferred in less than about 10 minutes.

### Method of the invention

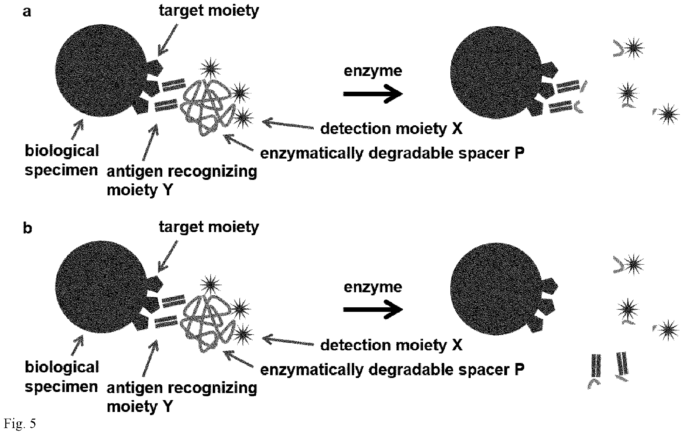

In another variant of the method of the invention, additionally the antigen recognizing moiety Y is removed from the target moiety after detection. The method may therefore involve a step d) wherein the enzymatically degradable spacer P is degraded by an enzyme, thereby removing the detection moiety X and the antigen recognizing moiety Y from the target moiety (Fig. 5 (b)).

This can be achieved by providing the conjugates comprising low-affinity antigen recognizing moieties Y. For most pMHC-complexes targeting TCR and/or KIR molecules the binding is characterized of being low-affin (equilibrium dissociation constant KD values of about 1E-07 M and larger, and /or dissociation rate constant k(off) values of about 1E-03 s-1 and larger). Such low-affinity antigen recognizing moieties do not provide a stable binding to the target moiety in a 1:1 ratio, but several low-affinity antigens recognizing moieties can be multimerized in one conjugate and therefore bind to the target moiety due to the avidity effect, i.e. m>=2 in formula (I). During the degradation of the enzymatically degradable spacer P, the degree of multimerization of the low-affinity antigen recognizing moieties in the conjugate is reduced, thereby initiating the dissociation of the antigen recognizing moieties. After dissociation of the low-affinity antigen recognizing moieties the target moiety is removed from the detection moiety X, from the spacer P and from the antigen recognizing moiety Y.

A preferred embodiment of the method of the invention comprises step d), in which the enzymatically degradable spacer P is degraded by an enzyme, thereby removing the detection moieties X from the target moiety.

In a variant of step d), the enzymatically degradable spacer P is degraded by an enzyme, thereby the detection moieties X and in addition also the antigen recognizing moieties Y are removed from the target moiety.

In a variant of the invention, the enzymatically degradable spacer P is degraded by an enzyme, thereby the detection moieties X and in addition also the antigen recognizing moieties Y are removed from the target moiety and also the linker units L and V from the target moiety.

The method of the invention may be performed as one or more sequences of steps a) to d). After each sequence, the detection moiety X and optionally the antigen recognizing moiety Y may be cleaved (removed) from the target moiety. Especially when the biological specimens are living cells which shall be further processed, the method of the invention has the advantage of providing unlabeled cells.

After and/or before each step a)-d), one or more washing steps may be performed to remove unwanted material such as unbound conjugate, parts of the conjugate such as free detection moiety X or free antigen recognizing moiety Y or reagents used for cleavage. The term "washing" as used herein means that the sample of biological specimen is separated from the environmental buffer by a suitable procedure, for example sedimentation, centrifugation, draining, or filtration. Before this separation step, washing buffer may be added and optionally incubated for a time period. After this separation step, the sample may be filled or resuspended again with buffer.

Any step a)-d) may be monitored qualitatively or quantitatively according to the detection moieties X used or by other applicable quantitative or qualitative methods known by persons skilled in the art. Examples comprise visual counting methods or flow cytometry analysis via labeling of the biological specimens with any cleavable- or non-cleavable fluorochrome conjugates, e.g. commercially available antibody-fluorochrome conjugates from Miltenyi Biotec, Thermo Fisher, Biolegend, or BD. This can be useful to determine the efficiency of the individual steps provided by the method of the invention.

### Step a)

In step a) of the method, at least one conjugate having the general formula (I) to (IV) is provided. In order to detect different target moieties or the same target moiety by different detection moieties, different conjugates having the general formula (I) to (IV) can be provided, wherein the conjugates and its components, Y, V, P, L, X, o, n, m, have the same meaning, but can be the same or different kind and/or amount of antigen recognizing moiety Y and/or linker V and/or enzymatically degradable spacer P and/or linker L and/or detection moiety X.

In further embodiments of the method additional conjugates different from the general formula (I) to (IV) of any kind for labeling a sample of biological specimen are provided. Said additional conjugates are known by person skilled in the art. Examples for said additional conjugates are conjugates that do not comprise an enzymatically degradable spacer P and will not be cleaved from a target moiety by step d) and comprise, for example, antibody conjugates targeting intracellular antigens such as transcription factors like FoxP3 as well as antigens on the surface of cells like CD3, CD4, CD8, CD14, CD19, CD20, CD25, CD40L, CD56, CD69, and CD154. A plurality of those conjugates like antibodies conjugated to fluorescent moieties or (magnetic) particles are commercially available from e.g. Miltenyi Biotec, Thermo Fisher, Biolegend, BD, or StemCell Technologies. Said additional conjugates may be used to label the sample of biological specimen in or after any of the steps a)-d) for qualitatively or quantitatively monitoring.

### Step b)

In step b) of the method, the target moiety of the sample of biological specimens is contacted and thereby labeled with the conjugate according to formula (I) to (IV).

In a variant of the invention with at least two different conjugates of the general formula (I) to (IV) targeting two different target moieties, contacting the sample specimens may be carried out either by adding said conjugates simultaneously or subsequently (by repeating step b)).

Furthermore, conjugates not bound to a target moiety may be removed by washing, for example with buffer, before the target moiety labeled with the conjugate is detected or isolated in step c) or before a next contacting step b).

In a variant of the invention, it is possible to perform multiple steps b).

Step b) may comprise at least one additional conjugate different from the general formula (I) to (IV) which can be incubated simultaneously at the same time point or with delay or subsequently in more than one step b).

Conditions of step b) during incubation are known by persons skilled in the art and may be empirically optimized in terms of time, temperature, pH, etc. Incubation time and incubation temperature is dependent on the binding strength of the conjugate to the target moiety and the application. Usually incubation time is up to 4 h, preferred up to 2 h, more preferred up to 1 h, and most preferred up to 30 min. Incubation temperature is usually in the range of 0 °C to 37 °C.

### Step c)

In step c) of the method, the target moiety labeled with a conjugate according to formula (I) to (IV) is detected with the detection moiety X. The method and equipment to detect the target moiety labeled with the conjugate is determined by the detection moiety X.

The method of the invention may be utilized not only for detecting target moieties i.e. target cells expressing such target moieties, but also for isolating the target cells from a sample of biological specimens according to the detection moiety X. In the method of the invention the term "detection" includes "isolation' as part of the detection process.

For example, the detection of a target moiety by fluorescence may be used to conduct an appropriate cell sorting process. In the method of the invention, the detection of a target moiety by a magnetic label may be used to conduct an appropriate cell separation process such as the well-known magnetic cell separation process. This process can also be used as detection and isolation process, wherein the magnetic particles are detected by the magnetic field.

In the invention, the detection moiety X is a fluorescent moiety. Targets labeled with the conjugate are detected by exciting the fluorescent moiety X and analyzing the resulting fluorescence signal. The wavelength of the excitation is usually selected according to the absorption maximum of the fluorescent moiety X and provided by LASER or LED sources as known in the art. If several different fluorescent moieties X are used for multiple color/parameter detection, care should be taken to select fluorescent moieties having not overlapping absorption and emission spectra, at least not overlapping absorption and emission maxima. The targets may be detected, e.g., under a fluorescence microscope, in a flow cytometer, a spectrofluorometer, or a fluorescence scanner. Light emitted by chemiluminescence can be detected by similar instrumentation omitting the excitation.

In another variant of the disclosure (not claimed) the detection moiety is a light absorbing moiety, which is detected by the difference between the irradiation light intensity and the transmitted or reflected light intensity. Light absorbing moieties might also be detected by photoacoustic imaging, which uses the absorption of a pulsed laser beam to generate an acoustic like an ultrasonic signal.

Radioactive detection moieties are detected though the radiation emitted by the radioactive isotopes. Suitable instrumentation for detection of radioactive radiation include, for example, scintillation counters. In case of beta emission electron microscopy can also be used for detection.

Transition metal isotope mass tag moieties are detected by mass spectrometric methods such as ICP-MS, which is integrated in mass cytometry instrumentation.

In a further variant of the disclosure (not claimed) the detection moiety is a solid support. Depending on the size and density those might be detected by visual inspection or in a microscope.

Magnetic particles are detected magnetically, e.g., by magnetic relaxometry, magnetic resonance imaging (MRI), magnetic force microscopy (MFM), superconducting quantum interference devices (SQUIDs), magnetometer.

The target moiety can be isolated according to their detection signal by optical means, electrostatic forces, piezoelectric forces, mechanical separation, acoustic means, or magnetic forces.

In one variant of the invention, suitable for such separations according to a fluorescence signal are especially flow sorters, e.g., FACS, TYTO^{®} or MEMS-based cell sorter systems, for example as disclosed in EP14187215.0 or EP14187214.3.

In another non-claimed variant, wherein the detection moiety is a solid support, the isolation may be performed by mechanical trapping of the solid support, e.g., in a column or a sieve, or according to their density, e.g. by sedimentation or centrifugation.

Furthermore, target moieties labeled with a magnetic particle may be isolated by applying a magnetic field. Magnetic cell sorting is known to the person skilled in the art and can be conducted in a permanent or an electromagnetic field with or without the use of a ferromagnetic column containing ferromagnetic material. Columns containing ferromagnetic material enhance the gradient of the magnetic field and are available from Miltenyi Biotec.

In further variants of the invention it is possible to combine at least two detection and/or isolation steps c) simultaneously or in subsequent steps.

Furthermore, during or after isolation of the target moieties, contaminating non-labeled moieties of the sample of biological specimen may be removed by washing, for example, with buffer.

### Step d)

After detection of the target moiety in step c), the spacer P is enzymatically degraded in step d) thereby cleaving at least the detection moiety X (including the optional linker L) from the target moiety. Step d) is optional and may be conducted if at least the detection moiety X and optionally also the antigen recognizing moiety Y should be cleaved from the target moiety.

Depending on the antigen recognizing moiety Y the method may involve a step d) wherein the enzymatically degradable spacer P is degraded by an enzyme, thereby removing the detection moiety X and the antigen recognizing moiety Y from the target moiety. In that variant, during the enzymatic cleavage of the spacer P the multimerization of low-affinity antigen recognizing moieties in the conjugate will be reduced and the antigen recognizing moieties may dissociate due to the low affinity which results in a complete cleavage of the detection moiety X (including the optional linker L), the spacer P, and the antigen recognizing moiety Y (including the optional linker V) from the target moiety.

Step d) may be performed inside or outside the detection system. Examples for a release outside the detection system comprise the incubation of fluorescently labeled target moieties in a tube after FACS sorting with an enzyme that degrades spacer P, and the incubation of magnetically separated cell with said enzyme outside the magnetic separation column in order to remove the magnetic label. Examples for a release inside the detection system comprise performing step d) during fluorescence microscopy, cytometry, photometry, or MRI. The reduction of the detection signal might therefore be monitored in real time. In another example the disruption may also take place within the magnetic field. The magnetically labeled target moiety can be unlabeled by adding, e.g. the competing molecule, to the column located in the magnetic field. In this variant, the target moieties are eluted from the column/the magnetic fields whereas the magnetic label remains on the column and in the magnetic field.

Optionally a further step c) can be performed directly after performing step d).

The detection moiety X, linker L, enzymatically degraded spacer P, antigen recognizing moiety Y, linker V and/or the reagent used for enzymatically degradation, as used or generated in step d) may be removed from the target cells. This may be performed by a washing step or by utilizing the methods described in step c). The removal may be achieved by mechanical trapping of the solid support, e.g., in a column or a sieve. Furthermore, magnetic particles as solid support may be removed by applying a magnetic field as already described for isolation of target moieties. Using ferromagnetic columns, this is preferably conducted in at least one (the same) or especially in two different columns containing ferromagnetic material. Conducting step c) as an isolation step for removal at least once provides a possibility to separate the released target moiety or to determine the efficiency of the disruption step d).

### Sequences of steps a) to d)

The method of the invention is especially useful for analysis, detection and/or isolation of specific target moieties present on target cells in or from complex mixtures and may be performed in one or more sequences of the steps a) to d). After each sequence, the detection moiety and optionally the antigen recognizing moiety Y may be cleaved from the target moiety. Furthermore, sequences with combinations of any of the steps a) to d) are possible. Sequences may be stopped at any of the steps a) to d). Additional washing steps may be performed.

In a variant of the invention, at least two conjugates may be provided simultaneously or in subsequent staining sequences, wherein each antigen recognizing moiety Y recognizes a different target moiety. In a further variant of the invention, at least two conjugates may be provided simultaneously or in subsequent staining sequences, wherein each conjugate comprises a different detection moiety X. In an alternative variant, at least two conjugates may be provided to the sample simultaneously or in subsequent staining sequences, wherein each conjugate comprises a different enzymatically degradable spacer P which is cleaved by different enzymes. In all variants, the labeled target moieties may be detected simultaneously or sequentially. Sequential detection may involve simultaneous enzymatical degradation of the spacer molecules P or subsequent enzymatical degradation of the spacer molecules P with optional intermediate removing (washing) of unbound moieties.

### Use of the Method

The method of the invention can be used for various applications in research, diagnostics and cellular therapy.

In a first use of the invention, biological specimens such as cells are detected and analyzed counting purposes. One example comprises the counting of cell numbers from a sample comprising a certain set of target moieties (antigens) recognized by the antigen recognizing moieties of the conjugates. The sample may comprise T cells with different TCRs. A pMHC conjugate specific for a certain TCR is added to specifically label and thereby to detect said target cell with said TCR. It is also possible to add a mixture of pMHC conjugates, each specific for a certain TCR. The usage of different detection moieties for each pMHC conjugate, for example different fluorophores, allows a discrimination of target cells. The number of labeled target cells is then analyzed by, for example, flow cytometry. The labeled target cells may also be sorted, for example by a flow sorter or by magnetic sorting in the case of a magnetic detection moiety. For a further usage, target cells may be unlabeled, i.e. the pMHC conjugate is degraded by addition of an enzyme that is capable to degrade the spacer P. As a consequence, at least the detection moiety is removed from target cells. When the binding of pMHC molecules to their specific TCRs is of low-affinity, they can also be removed from the target cells by dissociation and optional washing of the cells.

In a second use of the invention, one or more populations of biological specimens are separated for purification and isolation of target cells. Said isolated cells may be used in a plurality of downstream applications such as molecular diagnostics, cell cultivation, or immunotherapy. One example comprises the purification of antigen-specific T cells from peripheral blood mononuclear cells (PBMCs). A pMHC conjugate specific for a certain TCR, present on the antigen-specific T cell of interest, is added to PBMCs to specifically label and thereby to detect said target cell with said TCR. It is also possible to add a mixture of pMHC conjugates, each specific for a certain TCR. The labeled target cells are then be purified. In the case of a magnetic detection moiety (magnetic bead), the PBMCs labeled with the pMHC conjugate(s) are applied onto a column that is placed in a magnet. Cells that are bound to a pMHC conjugate with a magnetic bead are retained in the magnetic field in the column, while other cells are washed away. By removing the magnet, the labeled antigen-specific T cells are eluted. These cells can be analyzed. For a further usage, target cells may be unlabeled, i.e. the pMHC conjugate is degraded by addition of an enzyme that is capable to degrade the spacer P. As a consequence, at least the detection moiety (magnetic bead) is removed from target cells. When the binding of pMHC molecules to their specific TCRs is of low-affinity, they can also be removed from the target cells by dissociation and optional washing of the cells.

In another use of the invention, the molecular location of target moieties such as antigens on the biological specimens recognized by the antigen recognizing moieties of the conjugate is determined. Advanced imaging methods are known in the art, for example "Multi Epitope Ligand Cartography", "Chip-based Cytometry", and "Multioymx". Samples of biological specimen, for example cells or tissue comprising TCRs of interest embedded in paraffin, are contacted either once or in sequential cycles with pMHC conjugates. It is also possible to add a mixture of pMHC conjugates, each specific for a certain TCR. The usage of different detection moieties for each pMHC conjugate, for example different fluorophores, allows a discrimination of target cells. The location of the target moiety (the TCR of interest present on cells) is detected by the fluorescent moiety on the molecular level. For a sequential analysis of different TCRs, target cells are unlabeled, i.e. the pMHC conjugate is degraded by addition of an enzyme that is capable to degrade the spacer P. As a consequence, at least the detection moiety (fluorophore) is removed from target cells. When the binding of pMHC molecules to their specific TCRs is of low-affinity, they can also be removed from the target cells by dissociation and optional washing of the cells. Subsequent cycles of such a labeling-detection-removal procedure with at least one fluorescent moiety provide the possibility to map protein networks, to localize different cell types, and/or to analyze disease-related changes in the proteome.

In another use of the invention, NK cells in biological specimens are detected, analyzed and/or isolated using pMHC conjugates specific for KIR molecules present on target NK cells.

In another use of the invention, pMHC conjugates are used in *ex vivo* clinical applications. For example, isolated T cells from a patient are incubated *ex vivo* with a pMHC conjugate that specifically detects and binds certain antigen-specific T cells. Said target cells are then isolated, for example by magnetic separation. Afterwards, these antigen-specific T cells are expanded and administered to a patient.

### EXAMPLES

### Example 1: Cloning, expression and purification of recombinant pMHC molecules in E.coli

For the expression of recombinant MHC molecules in *E.coli*, synthetic genes encoding for the extracellular domains of α chain (also termed heavy chain) and β2-microglobulin (β2m) were each cloned into the bacterial expression plasmid pET9a separately.

A poly-histidine tag and optionally a C-terminal cysteine residue were genetically fused to the C-terminus of the extracellular domain of the A*0201 heavy chain, resulting in the expression plasmids pET9a-β2m, pET9a-A*0201-His, and pET9a-A*0201-His-Cys. After transformation into *E.coli* BL21(DE3), selected clones were grown in LB medium, and expression in shaker flasks was induced by the addition of IPTG. After 3 hours, cells were harvested. After cell disruption, inclusion bodies were isolated, washed, and then solubilized in 6 M GdnHCl, 20 mM Tris, pH 8. Solubilized inclusions bodies were centrifuged for 5 min, and the supernatant was used for refolding of the pMHC complex by rapid dilution. Therefore, β2m and chemically synthesized peptide pp65₄₉₅₋₅₀₃ (NLVPMVATV) were added to the refolding buffer (100mM Tris-HCl (pH 7.7), 400 mM L-arginine, 5 mM red. Glutathione (GSH), 0.5 mM ox. Glutathione (GSSG), 2 mM EDTA) at concentrations of 25 mg/L and 10 mg/L, respectively, and incubated for 2 hours at 4 °C. Then A*0201 heavy chain at a concentration of 30 mg/L was added and the refolding mix was further incubated for 96 h at 4°C. The pMHC complex was purified by anion exchange chromatography using an Äkta pure system, buffer A (20 mM Tris, 15 mM NaCl, pH 8) and buffer B (20 mM Tris pH 8.5, 1 M NaCl). The refolding mixture was diluted with buffer A until the conductivity was between 4-5 mS/cm. An anion exchange Q adsorber was assembled using two prefilters (0.45 µm and 0.22 µm MillexGV filter).

After equilibration with buffer A, the sample was loaded using a peristaltic pump with a flow rate of 40 ml/min. The next steps were performed via Äkta. The adsorber was connected and washed (without the prefilter systems) using 200 ml of buffer A with a flow rate of 4 ml/min. The elution was performed with a step-elution starting with 20% and then 40% up to 100% buffer B with 4 ml/min. Protein-containing fractions were monitored at 280 nm and collected. Table 1 shows an overview of purified pMHC complexes.

**Table 1: Yield and purity of purified pMHC complexes.**

| pMHC complex | Yield per L refolding | Yield [%] | Purity |
|---|---|---|---|
| A*0201-HIS/β2m/pp65₄₉₅₋₅₀₃ | 8.3 mg | 20.1 % | >90% |
| A*0201-HIS-Cys/β2m/pp65₄₉₅₋₅₀₃ | 8.9 mg | 21.5 % | >90% |

### Example 2: Generation of pMHC conjugates for reversible labeling of antigens.

Four pMHC complex conjugates A, B, C and D were prepared by chemical conjugation of said pMHC complexes to dextran and fluorescent dyes as described below. For functional analysis, pMHC molecule conjugates A, B, C and D were tested in cell surface labeling experiments with antigen-specific T cells as biological specimen.

PBMCs were expanded in the CliniMACS Prodigy^{®} using PepTivator CMV pp65, followed by Rapid Expansion Protocol using OKT3 CD3 antibody (30 ng/mL). The cells were then co-cultured at a ratio of 1:200 with irradiated HLA-A2+ feeder cells that had been loaded with 1 µg/mL pp65 peptide (instrument and reagents available from Miltenyi Biotec). Eight days later, cells were harvested, aliquoted, and cryopreserved. For labeling experiments, cells were thawed and cell surface labeling was performed. Antigen-specific T cells in PBS/EDTA/BSA buffer were stained for 15 min at room temperature with either pMHC molecule conjugate A, B, or C. After 10 min incubation, CD8-VioBlue^{®} (clone BW135/80, available from Miltenyi Biotec) was added and the cells were further incubated for 10 min at 4 °C. The cells were washed with cold PBS/EDTA/BSA buffer and analyzed by flow cytometry.

A first pMHC complex conjugate A was prepared. It comprises an enzymatically degradable spacer, wherein the pMHC molecules were "randomly-oriented" due to conjugation via naturally occurring lysine residues. The small organic molecule dye NHS-Vio667 (Miltenyi Biotec) was dissolved in DMSO and incubated with aminodextran (250 kDa and 50 amino groups/dextran, Fina Biosolutions) dissolved in PBS/EDTA buffer at concentration of 10 mg/mL in a molar ratio of aminodextran:NHS-Vio667 of 1:17.5. After 60 min incubation at room temperature, the dextran-Vio667 conjugate was purified via Amicon Centrifugal Filter Unit 10 kDa (Merck) with PBS/EDTA buffer. The amount of conjugated Vio667 was determined by the absorbance of the fluorescent dye at 647 nm. The degree of labeling of Vio667 per dextran was 15. The dextran-Vio667 conjugate was functionalized by incubation with SMCC (Thermo Fisher Scientific) for 60 min at room temperature at a concentration of 10 mg/mL in a molar ratio of dextran-Vio667:SMCC of 1:97.5. The functionalized dextran-Vio667 was purified by gel filtration with Sephadex-G25 (GE Healthcare) using PBS/EDTA buffer. To conjugate the pMHC complex A*0201-HIS/β2m/pp65₄₉₅₋₅₀₃, naturally occurring lysine residues were thiolized. Therefore, the pMHC complex was rebuffered into PBS/EDTA buffer via gel filtration with Sephadex-G25. Afterwards, the pMHC complex was incubated with 2-iminothiolane (Thermo Fisher Scientific) at a concentration of 1 mg/mL in a molar ratio of pMHC:2-iminothiolane of 1:10. After 60 min incubation at room temperature, the thiolized pMHC was purified via gel filtration with Sephadex-G25 utilizing PB S/EDTA buffer. For conjugation, the thiolized pMHC complex was added to functionalized dextran-Vio667 in a molar ratio of pMHC:dextran of 30:1. After 60 min incubation at room temperature, β-mercaptoethanol followed by N-ethylmaleimide were added sequentially with a molar excess to block unreacted maleimide- and thiol-functional groups. The pMHC complex conjugate A was purified by size exclusion chromatography using an Äkta pure system. Concentration of pMHC and dextran-Vio667 was determined by absorbances at 280 nm and 647 nm. The degree of labeling of pMHC per dextran-Vio667 was 22.

Conjugate A comprises the following units:
- enzymatically degradable spacer P = aminodextran
- no linker V
- no linker L
- Detection moiety X = Vio667
- pMHC complex is randomly conjugated, via the lysine residues

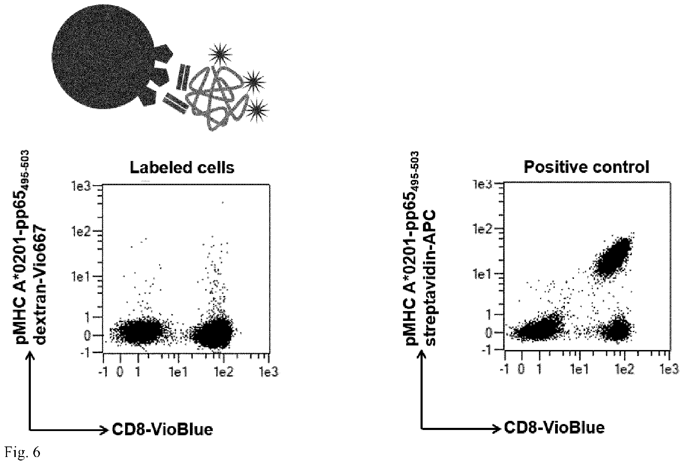

Fig. 6 shows exemplary dot plots of the result of flow cytometry analysis with pMHC complex conjugate A. For the analysis, a pregating on lymphocytes and an exclusion of dead cells by propidium iodide was conducted; target cells are CD8+ and pMHC+. No specific staining of either CD8+ or pMHC+ target cells was detectable. As positive control, a pMHC complex conjugate based on biotinylated pMHC complexes tetramerized via streptavidin-APC (available from, for example, MBL International Corporation) was used.

A second pMHC complex conjugate B was prepared. It also comprises an enzymatically degradable spacer, wherein the pMHC complex was covalently conjugated via its C-terminus. The small organic molecule dye NHS-Vio667 was dissolved in DMSO and incubated with aminodextran (250 kDa and 50 amino groups/dextran) dissolved in PBS/EDTA buffer at a concentration of 10 mg/mL in a molar ratio of aminodextran:NHS-Vio667 of 1:15. After 60 min incubation at room temperature, the dextran-Vio667 conjugate was purified via concentrator Amicon Centrifugal Filter Unit 10 kDa utilizing PBS/EDTA buffer. The amount of conjugated Vio667 was determined by absorbance at 647 nm. The degree of labeling of Vio667 per dextran was 10. The dextran-Vio667 conjugate was functionalized by incubation with SMCC for 60 min at room temperature at a concentration of 10 mg/mL in a molar ratio of dextran-Vio667:SMCC of 1:20. The functionalized dextran-Vio667 was purified by gel filtration with Sephadex-G25 utilizing PBS/EDTA buffer. To conjugate the pMHC complex A*0201-HIS-Cys/β2m/pp65₄₉₅₋₅₀₃ via its C-terminus, the pMHC complex was reduced by incubation with Tris(2-carboxyethyl)phosphin hydrochloride (TCEP HCl) (available from Thermo Fisher Scientific) at a concentration of 1 mg/mL in a molar ratio of pMHC:TCEP HCl of 1:5. After 30 min incubation at room temperature, the reduced pMHC complex was purified via gel filtration with Sephadex-G25 utilizing PBS/EDTA buffer. For conjugation, the reduced pMHC complex was added to functionalized dextran-Vio667 in a molar ratio of pMHC: dextran of 20: 1. After 60 min incubation at room temperature, β-mercaptoethanol followed by N-ethylmaleimide were added sequentially with a molar excess to block unreacted maleimide- or thiol-functional groups. The pMHC complex conjugate B was purified by size exclusion chromatography using an Äkta pure system. The concentration of pMHC and dextran-Vio667 was determined by absorbance at 280 nm and 647 nm. The degree of labeling of pMHC per dextran-Vio667 was 10.

Conjugate B comprises the following units:
- enzymatically degradable spacer P = aminodextran
- no linker V
- no linker L
- Detection moiety X = Vio667
- pMHC complex is conjugated via its C-terminus, with the Cys at the end

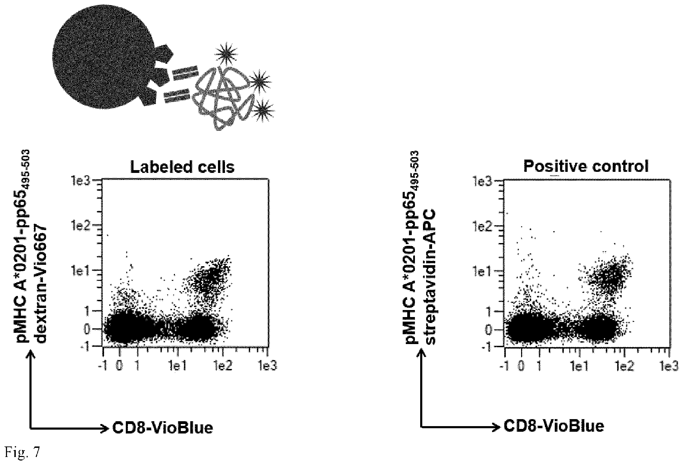

Fig. 7 shows exemplary dot plots of the result of flow cytometry analysis with pMHC complex conjugate B. For the analysis, a pregating on lymphocytes and an exclusion of dead cells by propidium iodide was conducted; target cells are CD8+ and pMHC+. In contrast to the result shown in Fig. 6 (conjugate A), CD8+ and pMHC+ target cells were specifically stained by conjugate B. As positive control, a pMHC complex conjugate based on biotinylated pMHC complexes tetramerized via streptavidin-APC (available from, for example, MBL International Corporation) was used. The examples demonstrate that for specific and stable binding of pMHC complex conjugates to target cells bearing TCR molecules it is crucial that the pMHC complex is conjugated covalently via its C-terminus to either a linker or a spacer, which is covalently connected to a detection moiety.

A third pMHC complex conjugate C was prepared. It also comprises an enzymatically degradable spacer, wherein the pMHC complex was covalently conjugated via its C-terminus and a linker V in a bivalent manner. The small organic molecule dye NHS-fluorescein (Thermo Fisher Scientific) was dissolved in DMSO and incubated with aminodextran (250 kDa and 50 amino groups/dextran) dissolved in PBS/EDTA buffer at a concentration of 10 mg/mL in a molar ratio of aminodextran:NHS-fluorescein of 1:25. After 60 min incubation at room temperature, the dextran-fluorescein conjugate was purified via concentrator Amicon Centrifugal Filter Unit 10 kDa utilizing PBS/EDTA buffer. The amount of conjugated fluorescein was determined by absorbance at 495 nm. The degree of labeling of fluorescein per dextran was 19. The dextran-fluorescein conjugate was functionalized by incubation with the multivalent linker MAL-L-Lys(MAL)-PEG4-TFP (Iris Biotec) for 60 min at room temperature at a concentration of 10 mg/mL in a molar ratio of dextran-fluorescein:MAL-L-Lys(MAL)-PEG4-TFP of 1: 15. The functionalized dextran-fluorescein was purified by gel filtration with Sephadex-G25 utilizing PBS/EDTA buffer. To conjugate the pMHC complex A*0201-HIS-Cys/β2m/pp65495-503 via its C-terminus, the pMHC was reduced by incubation with Tris(2-carboxyethyl)phosphin hydrochloride (TCEP HCl) (Thermo Fisher Scientific) at a concentration of 1 mg/mL in a molar ratio of pMHC:TCEP HCl of 1:5. After 30 min incubation at room temperature, the reduced pMHC complex was purified via gel filtration with Sephadex-G25 utilizing PBS/EDTA buffer. For conjugation, the reduced pMHC complex was added to functionalized dextran-fluorescein in a molar ratio of pMHC:dextran of 10:1. After 60 min incubation at room temperature, β-mercaptoethanol followed by N-ethylmaleimide were added sequentially with a molar excess to block unreacted maleimide- or thiol-functional groups. The pMHC complex conjugate D was purified by size exclusion chromatography using an Äkta pure system. The concentration of pMHC and dextran-fluorescein was determined by absorbance at 280 nm and 495 nm. The degree of labeling of pMHC per dextran-fluorescein was 7.

Conjugate C comprises the following units:
- enzymatically degradable spacer P = aminodextran
- linker V = MAL-L-Lys(MAL)-PEG4-TFP
- no linker L
- Detection moiety X = Fluorescein
- pMHC complex is conjugated via its C-terminus, with the Cys at the end

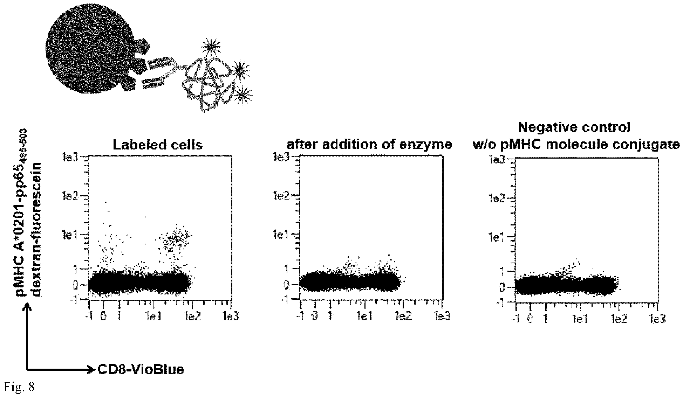

Fig. 8 shows exemplary dot plots of the result of flow cytometry analysis with pMHC complex conjugate C. For the analysis, a pregating on lymphocytes and an exclusion of dead cells by propidium iodide was conducted; target cells are CD8+ and pMHC+. CD8+ and pMHC+ target cells were specifically stained by conjugate C. After addition of the dextran-degrading enzyme dextranase, the remaining fluorescence intensity of the labeled target population was reduced down to the detection limit. The examples demonstrate that for specific and stable binding of pMHC complex conjugates to target cells bearing TCR molecules, in combination with a controllable removal of said pMHC complexes from said TCR molecules, it is crucial that the pMHC complex is conjugated covalently via its C-terminus to either a linker or a spacer, which is covalently connected to a detection moiety.

A fourth pMHC complex conjugate D was prepared. It also comprises an enzymatically degradable spacer, wherein the pMHC complex was covalently conjugated via its C-terminus and a linker V in a bivalent manner and wherein the detection moiety is covalently conjugated to the enzymatically degradable spacer P via the linker L. The small organic molecule dye NHS-PEG12-fluorescein (Iris Biotec) was dissolved in DMSO and incubated with aminodextran (250 kDa and 50 amino groups/dextran) dissolved in PBS/EDTA buffer at a concentration of 10 mg/mL in a molar ratio of aminodextran:NHS-PEG12-fluorescein of 1:25. After 60 min incubation at room temperature, the dextran-PEG12-fluorescein conjugate was purified via concentrator Amicon Centrifugal Filter Unit 10 kDa utilizing PBS/EDTA buffer. The amount of conjugated fluorescein was determined by absorbance at 495 nm. The degree of labeling of fluorescein per dextran was 23. The dextran-PEG12-fluorescein conjugate was functionalized by incubation with the multivalent linker MAL-L-Lys(MAL)-PEG4-TFP (Iris Biotec) for 60 min at room temperature at a concentration of 10 mg/mL in a molar ratio of dextran-PEG12-fluorescein:MAL-L-Lys(MAL)-PEG4-TFP of 1:15. The functionalized dextran-PEG12-fluorescein was purified by gel filtration with Sephadex-G25 utilizing PBS/EDTA buffer. To conjugate the pMHC complex A*0201-HIS-Cys/β2m/pp65495-503 via its C-terminus, the pMHC was reduced by incubation with Tris(2-carboxyethyl)phosphin hydrochloride (TCEP HCl) (Thermo Fisher Scientific) at a concentration of 1 mg/mL in a molar ratio of pMHC:TCEP HCl of 1:5. After 30 min incubation at room temperature, the reduced pMHC complex was purified via gel filtration with Sephadex-G25 utilizing PBS/EDTA buffer. For conjugation, the reduced pMHC complex was added to functionalized dextran-PEG12-fluorescein in a molar ratio of pMHC:dextran of 10:1. After 60 min incubation at room temperature, β-mercaptoethanol followed by N-ethylmaleimide were added sequentially with a molar excess to block unreacted maleimide- or thiol-functional groups. The pMHC complex conjugate D was purified by size exclusion chromatography using an Äkta pure system. The concentration of pMHC and dextran-PEG12-fluorescein was determined by absorbance at 280 nm and 495 nm. The degree of labeling of pMHC per dextran-fluorescein was 7.

Conjugate D comprises the following units:
- enzymatically degradable spacer P = aminodextran
- linker V = MAL-L-Lys(MAL)-PEG4-TFP
- linker L = PEG12
- Detection moiety X = Fluorescein
- pMHC complex is conjugated via its C-terminus, with the Cys at the end

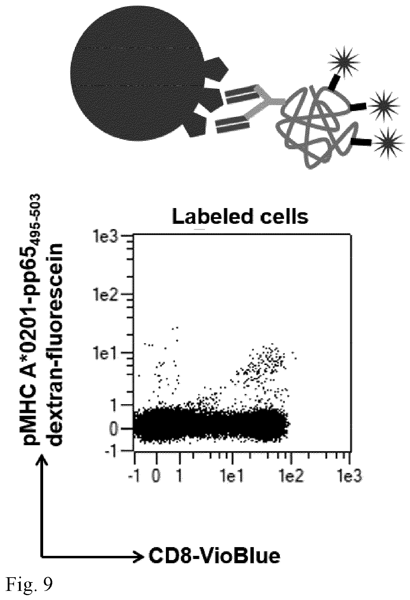

Fig. 9 shows exemplary dot plots of the result of flow cytometry analysis with pMHC complex conjugate D. For the analysis, a pregating on lymphocytes and an exclusion of dead cells by propidium iodide was conducted; target cells are CD8+ and pMHC+. CD8+ and pMHC+ target cells were specifically stained by conjugate D. The examples demonstrate that for specific and stable binding of pMHC complex conjugates to target cells bearing TCR molecules it is crucial that the pMHC complex is conjugated covalently via its C-terminus to either a linker or a spacer, which is covalently connected to a detection moiety.

## Claims

1. Conjugate for labelling a target moiety on a cell according to general formula (I)
(I) Xₒ-P- Yₘ
with Y : MHC-complex which is targeting TCR (T-cell receptor) and/or KIR (killer cell immunoglobulin-like receptor) molecules
P : enzymatically degradable spacer,
X : fluorescent dye,
o : integer between 5 and 25,
m: integer between 2 and 1000
wherein X and P; P and Y are covalently bound to each other **characterized in that** Y is bound to P via the C-terminus of Y.

2. Conjugate according to claim 1, **characterized in that** X is bound to P via linker L according to general formula (II) (Xₒ-L)ₙ - P - Yₘ
with L : linker unit,
n : integer between 1 and 1000
with the provisio that o * n <= 1000

3. Conjugate according to claim 1, **characterized in that** Y is bound to P via linker V according to general formula (III) Xₒ - P - (V- Yₘ)_{q}
with V : linker unit
q : integer between 2 and 1000.
with the provisio that q * m <= 1000

4. Conjugate according to claim 1, **characterized in that in that** X is bound to P via linker L and Y is bound to P via linker V according to general formula (IV) (Xₒ-L)ₙ - P - (V- Yₘ)_{q}
with V, L: same or different linker unit
q : integer between 2 and 1000.
n : integer between 1 and 1000.
with the provisios that o * n <= 1000 and q * m <= 1000

5. Conjugate according to claim 1, **characterized in that in that** X is bound to P via linker L and Y is bound to P via linker V according to general formula (V)
(Xₒ-L)ₙ (Xᵣ) P (Yₛ) (V- Yₘ)_{q}
with V, L: same or different linker unit
q : integer between 2 and 1000.
n : integer between 1 and 1000.
with the provisios that (o * n) + r <= 1000 and (q * m) + s <= 1000.

6. Conjugate according to any of the claims 1 to 5 **charaterized in that** the fluorescent dye X is selected from the group consisting of xanthene dyes, rhodamine dyes, coumarine dyes, cyanine dyes, pyrene dyes, oxazine dyes, pyridyl oxazole dyes, pyromethene dyes, acridine dyes, oxadiazole dyes, carbopyronine dyes, benzpyrylium dyes, fluorene dyes, fluorescent oligomers or fluorescent polymers.

7. Conjugate according to any of the claims 1 to 6 **charaterized in that** the enzymatically degradable spacer P is selected from the group consistiung of polysaccharides, proteins, peptides, depsipeptides, polyesters, nucleic acids, and derivatives thereof.

8. Conjugate according to any of the claims 2 to 7 **charaterized in that** the linker units V and L are selected from the group consisting of polyethylene glycol, peptides, proteins, polysaccharides, depsipeptides, polyesters, nucleic acids, polyvinylpyrrolidones, polyacrylates, polymethacrylates, polyoxazolines, polyvinyl alcohols, polyacrylamides, polycarbonates and derivatives thereof.

9. Method for detecting a target moiety in a sample of biological specimen by:
a) providing at least one conjugate having the general formula I
(I) Xₒ-P- Yₘ
with Y : MHC-complex which is targeting TCR (T-cell receptor) molecules
P : enzymatically degradable spacer,
X : fluorescent dye,
o : integer between 5 and 25,
m: integer between 2 and 1000
wherein X and P; P and Y are covalently bound to each other and Y is bound to P via the C-terminus of Y,
b) contacting the sample of biological specimens with the conjugate accoding to formula (I), thereby labeling the target moiety recognized by Y
c) detecting the target moiety labelled with the conjugate with the detection moiety X.

10. Method according to claim 9, **characterized in** providing at least one conjugate according to general formula (II) (Xₒ-L)ₙ - P - Yₘ wherein X is bound to P via linker L and
with L : linker unit,
n : integer between 1 and 1000
with the provisio that o * n <= 1000.

11. Method according to claim 9, **characterized in** providing at least one conjugate according to general formula (III) Xₒ - P - (V- Yₘ)_{q} wherein Y is bound to P via linker V and
with V : linker unit
q : integer between 2 and 1000.
with the provisio that q * m <= 1000.

12. Method according to claim 9, **characterized in** providing at least one conjugate according to general formula (IV) (Xₒ-L)ₙ - P - (V- Yₘ)_{q} wherein X is bound to P via linker L and Y is bound to P via linker V and
with V, L: same or different linker unit
q : integer between 2 and 1000.
n : integer between 1 and 1000.
with the provisios that o * n <= 1000 and q * m <= 1000.

13. Method according to claim 9, **characterized in** providing at least one conjugate according to general formula (V) (Xₒ-L)ₙ (Xᵣ) P (Yₛ) (V- Yₘ)_{q} wherein X is bound to P via linker L and Y is bound to P via linker V and
with V, L: same or different linker unit
q : integer between 2 and 1000.
n : integer between 1 and 1000.
with the provisios that (o * n) + r <= 1000 and (q * m) + s <= 1000.

14. Method according to any of the claims 9 to 13, **characterized in that** in a further step d), the enzymatically degradable spacer P is degraded by an enzyme, thereby cleaving the detection moieties X from the labelled target moiety.

15. Method according to any of the claims 9 to 13, **characterized in that** in a further step e), the enzymatically degradable spacer P is degraded by an enzyme, thereby cleaving the detection moieties from X and the antigen recognizing moieties Y from the labelled target moiety.

## Patentansprüche

1. Konjugat zur Markierung einer Zieleinheit auf einer Zelle gemäß der allgemeinen Formel (I)
(I) Xₒ-P-Yₘ
mit Y: MHC-Komplex, der auf TCR(T-Zell-Rezeptor)- und/oder KIR-Moleküle (Killerzell-Immunglobulin-ähnlicher Rezeptor) gerichtet ist,
P: enzymatisch abbaubarer Spacer,
X: Fluoreszenzfarbstoff,
∘: ganze Zahl zwischen 5 und 25,
m: ganze Zahl zwischen 2 und 1.000,
wobei X und P; P und Y kovalent aneinander gebunden sind, **dadurch gekennzeichnet, dass** Y über den C-Terminus von Y an P gebunden ist.

2. Konjugat nach Anspruch 1, **dadurch gekennzeichnet, dass** X über den Linker L gemäß der allgemeinen Formel (II) (Xₒ-L)ₙ-P-Yₘ, an P gebunden ist,
mit L: Linkereinheit,
n: ganze Zahl zwischen 1 und 1.000,
unter der Voraussetzung, dass o*n<=1.000.

3. Konjugat nach Anspruch 1, **dadurch gekennzeichnet, dass** Y über den Linker V gemäß der allgemeinen Formel (III) Xₒ-P-(V-Yₘ)_{q} an P gebunden ist,
mit V: Linkereinheit,
q: ganze Zahl zwischen 2 und 1.000,
unter der Voraussetzung, dass q*m<=1.000.

4. Konjugat nach Anspruch 1, **dadurch gekennzeichnet, dass** X über den Linker L an P gebunden ist und Y über den Linker V gemäß der allgemeinen Formel (IV) (Xₒ-L)ₙ-P-(V-Yₘ)_{q} an P gebunden ist, mit V,L: gleiche oder verschiedene Linkereinheit,
q: ganze Zahl zwischen 2 und 1.000,
n: ganze Zahl zwischen 1 und 1.000,
unter der Voraussetzung, dass o*n<=1.000 und q*m<=1.000.

5. Konjugat nach Anspruch 1, **dadurch gekennzeichnet, dass** X über den Linker L an P gebunden ist und Y über den Linker V gemäß der allgemeinen Formel (V) an P gebunden ist
(Xₒ-L)ₙ(Xᵣ)P(Yₛ)(V-Yₘ)_{q}
mit V, L: gleiche oder verschiedene Linkereinheit,
q: ganze Zahl zwischen 2 und 1.000,
n: ganze Zahl zwischen 1 und 1.000,
unter der Voraussetzung, dass (o*n)+r <= 1000 und (q*m)+s <= 1000.

6. Konjugat nach einem der Ansprüche 1 bis 5, **dadurch gekennzeichnet, dass** der Fluoreszenzfarbstoff X ausgewählt ist aus der Gruppe bestehend aus Xanthen-Farbstoffen, Rhodamin-Farbstoffen, Cumarin-Farbstoffen, Cyanin-Farbstoffen, Pyren-Farbstoffen, Oxazin-Farbstoffen, Pyridyl-Oxazol-Farbstoffen, Pyromethen-Farbstoffen, Acridin-Farbstoffen, Oxadiazol-Farbstoffen, Carbopyronin-Farbstoffen, Benzpyrylium-Farbstoffen, Fluoren-Farbstoffen, Fluoreszenz-Oligomeren oder Fluoreszenz-Polymeren.

7. Konjugat nach einem der Ansprüche 1 bis 6, **dadurch gekennzeichnet, dass** der enzymatisch abbaubare Spacer P ausgewählt ist aus der Gruppe bestehend aus Polysacchariden, Proteinen, Peptiden, Depsipeptiden, Polyestern, Nukleinsäuren und Derivaten davon.

8. Konjugat nach einem der Ansprüche 2 bis 7, **dadurch gekennzeichnet, dass** die Linkereinheiten V und L ausgewählt sind aus der Gruppe bestehend aus Polyethylenglykol, Peptiden, Proteinen, Polysacchariden, Depsipeptiden, Polyestern, Nukleinsäuren, Polyvinylpyrrolidonen, Polyacrylaten, Polymethacrylaten, Polyoxazolinen, Polyvinylalkoholen, Polyacrylamiden, Polycarbonaten und Derivaten davon.

9. Verfahren zur Detektion einer Zieleinheit in einer Probe eines biologischen Präparats durch:
a) Bereitstellen mindestens eines Konjugats der allgemeinen Formel I
(I) Xₒ-P-Yₘ
mit Y: MHC-Komplex, der auf TCR (T-Zell-Rezeptor)-
Moleküle gerichtet ist,
P: enzymatisch abbaubarer Spacer,
X: Fluoreszenzfarbstoff,
∘: ganze Zahl zwischen 5 und 25,
m: ganze Zahl zwischen 2 und 1.000,
wobei X und P; P und Y kovalent aneinander gebunden sind und Y über den C-Terminus von Y an P gebunden ist,
b) Inkontaktbringen der Probe der biologischen Präparate mit dem Konjugat gemäß Formel (I), wodurch die von Y erkannte Zieleinheit markiert wird,
c) Nachweisen der mit dem Konjugat markierten Zieleinheit mit der Nachweiseinheit X.

10. Verfahren nach Anspruch 9, das durch das Bereitstellen mindestens eines Konjugats gemäß der allgemeinen Formel (II) (Xₒ-L)ₙ-P-Yₘ gekennzeichnet ist, wobei X über einen Linker L an P gebunden ist, und
mit L: Linkereinheit,
n: ganze Zahl zwischen 1 und 1.000,
unter der Voraussetzung, dass o*n<=1.000.

11. Verfahren nach Anspruch 9, **dadurch gekennzeichnet, dass** mindestens ein Konjugat gemäß der allgemeinen Formel (III) Xₒ-P-(V-Yₘ)_{q} bereitgestellt wird, wobei Y über den Linker V an P gebunden ist, und
mit V: Linkereinheit,
q: ganze Zahl zwischen 2 und 1.000,
unter der Voraussetzung, dass q*m <= 1.000.

12. Verfahren nach Anspruch 9, **dadurch gekennzeichnet, dass** mindestens ein Konjugat gemäß der allgemeinen Formel (IV) (Xₒ -L)ₙ-P-(V-Yₘ)q bereitgestellt wird, wobei X über den Linker L an P gebunden ist und Y über den Linker V an P gebunden ist und
mit V, L: gleiche oder verschiedene Linkereinheit,
q: ganze Zahl zwischen 2 und 1.000,
n: ganze Zahl zwischen 1 und 1.000,
unter der Voraussetzung, dass o*n<=1.000 and q*m <= 1.000.

13. Verfahren nach Anspruch 9, **dadurch gekennzeichnet, dass** mindestens ein Konjugat gemäß der allgemeinen Formel (V) (Xₒ -L)ₙ (Xᵣ₎ P(Yₛ)(V-Y)_{mq} bereitgestellt wird, wobei X über den Linker L an P gebunden ist und
Y über den Linker V an P gebunden ist und
mit V, L: gleiche oder verschiedene Linkereinheit,
q: ganze Zahl zwischen 2 und 1.000,
n: ganze Zahl zwischen 1 und 1.000,
unter der Voraussetzung, dass (o*n)+r <=1.000 und (q*m)+s <=1.000.

14. Verfahren nach einem der Ansprüche 9 bis 13, **dadurch gekennzeichnet, dass** in einem weiteren Schritt d) der enzymatisch abbaubare Spacer P durch ein Enzym abgebaut wird, wodurch die Nachweiseinheiten X von der markierten Zieleinheit abgespalten werden.

15. Verfahren nach einem der Ansprüche 9 bis 13, **dadurch gekennzeichnet, dass** in einem weiteren Schritt e) der enzymatisch abbaubare Spacer P durch ein Enzym abgebaut wird, wodurch die Nachweiseinheiten von X und die Antigen erkennenden Einheiten Y von der markierten Zieleinheit abgespalten werden.

## Revendications

1. Conjugué pour marquer une fraction cible sur une cellule selon la formule générale (I)
(I) Xₒ-P-Yₘ
avec Y : complexe-MHC qui cible des molécules TCR (récepteur de lymphocyte T) et/ou KIR (récepteur de type immunoglobuline de cellule tueuse)
P: espaceur enzymatiquement dégradable,
X: colorant fluorescent,
o : nombre entier compris entre 5 et 25,
m: nombre entier compris entre 2 et 1 000
X et P ; P et Y étant liés de manière covalente l'un à l'autre **caractérisé en ce que** Y est lié à P via l'extrémité C-terminale de Y.

2. Conjugué selon la revendication 1, **caractérisé en ce que** Y est lié à P via un lieur L selon la formule générale (II) (Xₒ-L)ₙ-P-Yₘ
avec L : unité de lieur,
n: nombre entier compris entre 1 et 1 000
à la condition que o*n <= 1 000.

3. Conjugué selon la revendication 1, **caractérisé en ce que** Y est lié à P via un lieur V selon la formule générale (III) Xₒ-P-(V-Yₘ)_{q}
avec V : unité de lieur
q : nombre entier compris entre 2 et 1 000
à la condition que q*m <= 1 000.

4. Conjugué selon la revendication 1, **caractérisé en ce que** X est lié à P via le lieur L et Y est lié à P via le lieur V selon la formule générale (IV) (Xₒ-L)ₙ-P-(V-Yₘ)_{q}
avec V, L : unité de lieur identique ou différente
q : nombre entier compris entre 2 et 1 000
n : nombre entier compris entre 1 et 1 000
aux conditions que o*n <= 1 000 et q*m <= 1 000.

5. Conjugué selon la revendication 1, **caractérisé en ce que** X est lié à P via le lieur L et Y est lié à P via le lieur V selon la formule générale (V)
(Xₒ-L)ₙ(Xᵣ) P (Yₛ) (V-Yₘ)_{q}
avec V ; L : unité de lieur identique ou différente
q : nombre entier compris entre 2 et 1 000
n : nombre entier compris entre 1 et 1 000
aux conditions que (o*n) + r <= 1 000 et (q*m) + s <= 1 000.

6. Conjugué selon l'une quelconque des revendications 1 à 5 **caractérisé en ce que** le colorant fluorescent X est choisi dans le groupe constitué par les colorants xanthène, les colorants rhodamine, les colorants coumarine, les colorants cyanine, les colorants pyrène, les colorants oxazine, les colorants oxazole de pyridyle, les colorants pyrométhène, les colorants acridine, les colorants oxadiazole, le colorants carbopyronine, le colorants benzpyrilium, les colorants fluorène, les oligomères fluorescents ou les polymères fluorescents.

7. Conjugué selon l'une quelconque des revendications 1 à 6 **caractérisé en ce que** l'espaceur enzymatiquement dégradable P est choisi dans le groupe constitué par les polysaccharides, les protéines, les peptides, les depsipeptides, les polyesters, les acides nucléiques et leur dérivés.

8. Conjugué selon l'une quelconque des revendications 2 à 7 **caractérisé en ce que** les unités de lieur V et L sont choisies dans le groupe constitué par le polyéthylène glycol, les peptides, les protéines, les polysaccharides, les depsipeptides, les polyesters, les acides nucléiques, les polyvinylpyrrolidones, les polyacrylates, les polyméthacrylates, les polyoxazolines, les alcools polyvinyliques, les polyacrylamides, les polycabonates et leurs dérivés.

9. Procédé de détection d'une fraction cible dans un échantillon de spécimen biologique en :
a) fournissant au moins un conjugué ayant la formule générale (I)
(I) Xₒ-P-Yₘ
avec Y : un complexe-MHC qui cible des molécules de TCR (récepteur de lymphocyte T)
P : espaceur enzymatiquement dégradable,
X : colorant fluorescent,
o : nombre entier compris entre 5 et 25,
m : nombre entier compris entre 2 et 1 000
X et P ; P et Y étant liés de manière covalente l'un à l'autre et Y étant lié à P via l'extrémité C-terminale de Y,
b) la mise en contact de l'échantillon de spécimens biologiques avec le conjugué selon la formule (I), marquant ainsi la fraction cible reconnue par Y
c) la détection de la fraction cible marquée avec le conjugué avec la fraction de détection X.

10. Procédé selon la revendication 9, **caractérisé** comme fournissant au moins un conjugué selon la formule générale (II) (Xₒ-L)ₙ-P-Yₘ X étant lié à P via le lieur L et
avec : L : unité de lieur
n : nombre entier compris entre 1 et1 000
à la condition que o*n <= 1 000.

11. Procédé selon la revendication 9, caractérisé comme fournissant au moins un conjugué selon la formule générale (III) Xₒ-P-(V-Yₘ)_{q} Y étant lié à P via le leur V et
avec V : unité de lieur
q : nombre entier compris entre 2 et 1 000
à la condition que q*m <= 1 000.

12. Procédé selon la revendication 9, caractérisé comme fournissant au moins un conjugué selon la formule générale (IV) (Xₒ-L) ₙ-P-(V-Yₘ)_{q} X étant lié à P via le lieur L et Y étant lié à P via le lieur V et
avec : V,L : unité de lieur identique ou différente
q : nombre entier compris entre 2 et 1 000
n : nombre entier compris entre 1 et 1 000
aux conditions que o*n <= 1 000 et q*m <= 1 000.

13. Procédé selon la revendication 9, caractérisé comme fournissant au moins un conjugué selon la formule générale (V) (Xₒ-L) ₙ (Xᵣ) P (Yₛ) (V-Yₘ)_{q} X étant lié à P via le lieur L et Y étant lié à P via le lieur V et avec V, L : unité de lieur identique ou différente q : nombre entier compris entre 2 et 1 000 n : nombre entier compris entre 1 et 1 000 aux conditions que (o*n) + r <= 1 000 et (q*m) + s <= 1 000.

14. Procédé selon l'une quelconque des revendications 9 à 13, **caractérisé en ce que** dans une autre étape d), l'espaceur enzymatiquement dégradable P est dégradé par une enzyme, clivant ainsi les fractions de détection X de la fraction cible marquée.

15. Procédé selon l'une quelconque des revendications 9 à 13, **caractérisé en ce que** dans une autre étape e), l'espaceur enzymatiquement dégradable P est dégradé par une enzyme, clivant ainsi les fractions de détection X et les fractions Y reconnaissant l'antigène de la fraction cible marquée.
